# EUROPEAN PATENT SPECIFICATION

(11) **EP 4 427 776 B1**
(45) Date of publication and mention of the grant of the patent: **07.10.2026**
(21) Application number: 24190679.1
(22) Date of filing: 01.06.2017
(51) Int. Cl.: A61M 5/32

(54) **THREE POSITION NEEDLE RETRACTION**
NADELRÜCKZUG MIT DREI POSITIONEN
RÉTRACTION D'AIGUILLE À TROIS POSITIONS

(30) Priority: 02.06.2016 US 201662344782 P
(43) Date of publication of application: 11.09.2024
(62) Document number(s) of the earlier application(s) in accordance with Art. 76 EPC: 17729699.3 / 3 463 526
(73) Proprietor: West Pharma Services IL, Ltd, 4366411 Ra'anana (IL)
(72) Inventor: YUDELEVICH, Michael, Winnipeg, Manitoba, R2C 2P8 (CA); BAR-EL, Yossi, 71947 Beit Arye (IL); YIGAL, Gil, 70800 Gan Yavne (IL); FILMAN, Reuven, Y., 42560 Netanya (IL)
(74) Representative: Petraz, Gilberto Luigi

(56) References cited:
- WO-A1-2011/090955
- WO-A2-2004/069302
- US-A1- 2002 055 711

## Description

### RELATED APPLICATION/S

### FIELD AND BACKGROUND OF THE 2INVENTION

The present invention, in some embodiments thereof, relates to a needle extension and/or retraction mechanism and, more particularly, but not exclusively, to a mechanism for protecting a needle tip from stick hazards after removal of an autoinjector for example a patch injector from a subject.

U.S. Patent no. 8915882 relates to "A needle assembly adapted for fluid communication with a vial containing a substance to be delivered to a subject, the needle assembly including a needle held in a needle holder, the needle holder confined to move in a housing, and an activation mechanism for activating delivery of the substance through the needle, the activation mechanism including a safety latch that initially impedes movement of the needle holder, wherein when the safety latch is placed on the subject, the safety latch moves to a position that permits moving the needle holder to cause the needle to protrude outwards of the housing to pierce the subject to allow administration of the substance to the subject."

U.S. Patent no. 8152779 relates to "A needle assembly adapted for fluid communication with a cartridge containing a substance to be delivered to a subject, the needle assembly characterized by a biasing device (70) arranged to apply a biasing force on a needle (28, 116) to cause the needle (28, 116) to protrude outwards of a housing (30, 112) to pierce the subject, and biasing device release apparatus (60) including a biasing device arrestor (62) that initially blocks movement of the biasing device (70) and which releases the biasing device (70) when the safety latch (32, 122) moves to a position that permits moving the needle holder (26, 118) to cause the needle (28, 116) to protrude outwards of the housing (30, 112)."

U.S. Patent no. 7530964, corresponding to the application published under the publication number US 2002/055711, relates to a needle device that, "has a needle retraction mechanism that retracts the needle upon removing the device from the skin surface (either intentionally or unintentionally). Once the needle is retracted, the device is rendered inoperative. The needle can be further made inoperative by bending it when one attempts to reuse the device. In another embodiment, a needle opening formed in the base of the housing can be covered to render the needle inoperative when one attempts to reuse the device. In another embodiment, the needle device instead has a needle shield that automatically covers the needle after use."

U.S. Patent no. 9072827 relates to a method and device "for preventing a needle stick hazard in the event of a collapse of a protective needle flap of a portable drug pump. The device may include a needle guide, a secure space and/or a shield. A point of a needle is optionally deflected into a secure space upon collapse of the protective flap. The space may optionally be shielded. Optionally, the support linking the needle to the pump may pivot and/or translate. Optionally, there may be an exposing position wherein the needle protrudes through an opening in the flap. Optionally, the opening may be non-circular."

### SUMMARY OF THE INVENTION

The invention is according to claim 1. Preferred embodiments of the invention are set forth in the dependent claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
*FIG. 1* is a flow chart illustration of a method, which is not part of the invention, of partially retracting a needle in accordance with an embodiment of the current invention;
*FIG. 2* is a flow chart illustration of a method of shielding and retracting a needle, which is not part of the invention, in accordance with an embodiment of the current invention;
*FIG. 3* is a flow chart illustration of a method of using and retracting a needle, which is not part of the invention, in accordance with an embodiment of the current invention;
*FIG. 4* is a state diagram illustration of a partially retractable needle in accordance with an embodiment of the current invention;
*FIG. 5* is a state diagram illustration of a needle in accordance with an embodiment of the current invention;
*FIG. 6* is a state diagram illustration of a multi-position needle in accordance with an embodiment of the current invention;
*FIG. 7* is a block diagram illustration of a partially retractable needle in accordance with an embodiment of the current invention;
*FIG. 8* is a block diagram illustration of a needle mechanism in accordance with an embodiment of the current invention;
*FIG. 9* is a perspective cutaway cross sectional illustration of a multistate needle insertion and protection system in a preactivated state in accordance with an embodiment of the current invention;
*FIG. 10* is a perspective cutaway cross sectional illustration of a mechanism holding a needle in a preactivated state in accordance with an embodiment of the current invention;
*FIGs. 11A-11C* are a perspective cutaway and cross sectional illustrations of a needle mechanism in a primed state in accordance with an embodiment of the current invention;
*FIGs. 12A-12D* are a perspective cutaway and cross sectional illustrations of a needle mechanism in an active state in accordance with an embodiment of the current invention;
*FIGs. 13A-13C* are perspective cutaway illustrations of a mechanism holding a needle in an intermediate state in accordance with an embodiment of the current invention;
*FIG. 14* is a perspective cutaway illustration of a mechanism holding a needle in a retracted state in accordance with an embodiment of the current invention;
*FIG. 15* is a perspective cutaway illustration of a mechanism in an activated state in accordance with an embodiment of the current invention;
*FIG. 16* is a perspective cutaway illustration of a mechanism holding a needle in an intermediate state in accordance with an embodiment of the current invention;
*FIG. 17* is a perspective cutaway illustration of a mechanism in an active state in accordance with an embodiment of the current invention;
*FIG. 18* is a perspective cutaway illustration of a mechanism holding a needle in an intermediate state in accordance with an embodiment of the current invention;
*FIG. 19* is a perspective cutaway illustration of a mechanism holding a needle in an intermediate state in accordance with an embodiment of the current invention;
*FIG. 20* is a schematic illustration of dual rotating needle shields in accordance with an embodiment of the current invention;
*FIG. 21A and 21B* are schematic illustrations of a combination of rotating and translating needle shields in accordance with an embodiment of the current invention;
*FIG. 22* is a schematic illustration of a needle protection latch in accordance with an embodiment of the current invention;
*FIG. 23* is a schematic illustration of a translating needle shield in accordance with an embodiment of the current invention; and
*FIGs. 24A-24C* are a schematic illustrations of a needle protection mechanism in accordance with an embodiment of the current invention;
*FIGs. 25A and 25B* illustrate a sliding needle shield in accordance with an embodiment of the current invention;
*FIG. 26A* is a perspective view of a needle mechanism in a preactivated locked state in accordance with an embodiment of the current invention;
*FIG. 26B* is a perspective view of a needle mechanism in a primed unlocked state in accordance with an embodiment of the current invention;
*FIG. 26C* is a cutaway view of a needle mechanism in accordance with an embodiment of the current invention;
*FIGs. 27A to 27C* illustrate a needle assembly in a primed state in accordance with an embodiment of the current invention;
*FIGs. 28A to 28C* illustrate a needle assembly in an activated state in accordance with an embodiment of the current invention;
*FIGs. 29A-29C* illustrate a needle retraction mechanism in a partially protected state in accordance with an embodiment of the current invention and
*FIGs. 30A-30C* illustrate a needle retraction mechanism in a fully protected state in accordance with an embodiment of the current invention.

### DESCRIPTION OF SPECIFIC EMBODIMENTS OF THE INVENTION

The present invention, in some embodiments thereof, relates to a needle extension and/or retraction mechanism and, more particularly, but not exclusively, to a mechanism for protecting a needle tip from stick hazards after removal of an autoinjector for example a patch injector from a subject.

### OVERVIEW

An aspect of some embodiments of the present invention relates to an autoinjector with a needle that automatically partially retracts when the injector is removed from the skin of a subject. In some embodiments, an injector needle extends to an active position with an exposed portion of the needle extending outward from a skin contact surface and/or from an opening of a base of a housing of the injector. Optionally, in the partially retracted state a smaller portion of the needle extends outward from the skin contact surface and/or the opening. Optionally the smaller portion of the needle is covered by a shield for example to avoid a stick hazard.

In some embodiments a sensor may be stimulated by an external event, for example, a sensor registers when the injector is placed onto and/or distanced from the skin of a subject. Optionally, sensor output triggers needle retraction and/or unlocks the needle from the extended position. For example, the sensor may include a mechanical component in contact with the skin and/or mechanically interconnected to a needle retraction mechanism and/or a needle locking mechanism. For example, the mechanical connection may lock the needle in the extended position when the sensor is against the skin. Distancing a portion of the injector from the skin optionally releases the mechanical interconnection to unlock the needle and/or to trigger partial retraction of the needle.

In some embodiments, reexposure of a needle is inhibited. For example, after retraction, a locking mechanism may prevent the needle from being reextended. Alternatively or additionally, a shield may be deployed blocking the needle. Optionally the shield may be locked in place preventing reexposure of the needle. Alternatively or additionally, further excitation of the sensor (for example moving the sensor) may cause and/or allow further retraction of the needle to a protected location. Alternatively or additionally, a needle may retract, lock and/or unlock in connection to an event and/or status of the injector, for example, when all of a payload has been discharged and/or when there is an occlusion in the system and/or in response to a user action and/or after a time lapse.

In some embodiments a device may include a needle retraction mechanism and/or a needle shield. For example, the skin sensor may be designed to shield the needle and/or prevent a stick hazard. Optionally the shield maybe deployed when delivery finishes and/or when the device is distanced from an injection surface (e.g. the skin of a user). Optionally or additionally, the shield may protect the needle when the needle is in an intermediate position. For example the shield and/or sensor may have the form of a flap covering the needle point and/or a sleeve surrounding the needle point.

In some embodiments a needle may be held in a partially retracted position for a time period. For example, the needle may be held in the partially retracted position for between 0.1 sec to 1 sec and/or between 1 sec to 5 sec and/or between 5 sec. to 20 sec and/or longer. Alternatively or additionally, the needle may be held until an external stimulus causes it to be released. For example the external stimulus may include a force exerted on an element of the drug delivery device. For example the needle may be released in response to a force on a needle shield and/or a force on the needle and/or a movement of the needle shield.

In some embodiments, a needle may be retracted from an extended position to an intermediate position and/or a fully retracted position in response to an external stimulus. For example, the needle may be retracted in response to the delivery device being distanced from an injection surface (for example the skin of a subject). For example the needle may be retracted in response to removal of a skin contact surface of the device from the injection surface. For example the needle may be retracted in response to an action of a user, for example pushing a retraction button. Alternatively or additionally the needle may be removed in response to a status of the delivery device. For example, the needle may be retracted when the device completes delivery of a prescribed dose and/or upon a malfunction of the device.

An aspect of some embodiments of the present invention relates to a multi-level safety mechanism to protect a needle. In some embodiments, the safety mechanism includes a first component that protects the needle and a second component that is activated in response to changes in the first component. For example, a needle shield in a deployed configuration may block a sharp tip of the needle. When the needle shield is compromised, for example, a force is applied to the shield and/or the shield moves from the deployed configuration, the needle is optionally neutralized. For example the needle may be retracted and/or folded and/or moved to a protected position.

In some embodiments a mount connecting a needle to a drug delivery device may include a needle retraction system and/or multiple protection measures and/or a redundant needle protection system. Optionally, the needle mount may allow movement of the needle with respect to the housing. For example, the housing may include a skin contact surface and/or a needle opening in the skin contact surface. Optionally, the needle is mounted to the housing to allow movement of needle in a longitudinal directional and/or movements that are nearly longitudinal (for example around a pivot approximately perpendicular to and/or far from the needle [for example the distance to the fulcrum may be more than 4 times the length of the needle]). Optionally, a point of the needle moves from a location inside the housing to a location outside the housing. For example, the needle point may pass through the needle opening. Optionally the housing may include a skin contact surface. In some embodiments, the needle opening may pass through the skin contact surface. Optionally the skin contact surface includes an adhesive for attachment to an injection site on a subject. In some embodiments, the needle is hollow. For example, an end of the needle opposite the tip may be connected to a drug reservoir. For example, the needle may be extended into the skin of a subject and/or the needle may act as a fluid path for injecting the drug from the reservoir into the subject.

In some embodiments, an interconnection between a needle shield and a retraction mechanism may lock the needle in the extended and/or partially retracted position. For example, the needle may be locked in the extended and/or partially retracted position while the needle shield is deployed. Movement of the needle shield may release the lock and allow the needle to be further retracted. In some embodiments, the needle may retract automatically. Alternatively or additionally, the needle may be unlocked and/or retract in response to an external force. For example, the needle shield may be interlocked with the needle retraction mechanism such that a force on the needle shield is translated to a force pulling back the needle to a retracted and/or protected position. Alternatively or additionally, the needle shield may have a natural path of movement, for example a path of least resistance to movement that intersects the needle. Movement of the shield along the path optionally causes the shield to contact the needle. Contact between the shield and the needle optionally neutralizes the needle, for example by bending the needle and/or pushing it into a protected position.

An aspect of some embodiments of the current invention relates to a needle having 3 or more stable positions. For example, a needle may have a retracted position, an extended position and/or third position. For example, the third position may include a partially retracted position and/or an intermediate position between the extended and retracted positions. Optionally, in the partially retracted position the needle may be entirely retracted into a housing of the device. Alternatively or additionally, in the partially retracted position, a portion of the needle may extend out of the housing. For example the portion of the needle extending out of the housing in the partially retracted position may be shorter than the portion extending out in the extended position.

In some embodiments, a needle retraction mechanism may be locked and/or unlocked and/or triggered by a skin sensor. Optionally, the skin sensor may be mechanically interlocked with the needle retraction mechanism. For example, when the sensor detects removal from the skin, the needle may be unlocked from an extended position. Alternatively or additionally, when the sensor detects removal from the skin, the needle may be automatically retracted to a partially and/or fully retracted position. For example, when the sensor detects removal from the skin the needle retraction mechanism may be moved to and/or locked to a partially and/or fully retracted position. Alternatively or additionally, other events may lock, unlock and/or automatically move the needle retraction mechanism between positions.

In some embodiments, in an active state, a sharp needle and/or cannula may extend out of the device. In a safeguarded state, the needle is optionally partially retracted. For example, in the extended state a portion of the needle may be exposed and/or extend outside a housing of the device. Optionally, in the partially retracted state, a smaller portion of the needle may be exposed and or extend out of the device than in the extended state. For example, in the extended state a needle tip may extend a distance away from a housing of the device. Optionally, in the partially retracted state, the needle may extend away from the device less than in the exposed state. Optionally, the device may have a fully retracted state and an intermediate state. In the intermediate state the needle may be on a path between the fully retracted state and the extended state. For example, during injection a needle may extend a distance ranging for example between 2 to 5 mm and/or 5 to 8 mm and/or 8 to 12 mm out of the skin contact surface. In the partially retracted state a needle may extend a distance ranging between 20 to 70% and/or 70 to 85% and/or 85 to 95% of the fully extended distance. Optionally, in the partially retracted state the point of the needle may be located ranging for example between 0.5 to 1 mm and/or 1mm to 3 mm and/or 3 mm to 5 mm and/or 5 to 10 mm outward from the position in the fully retracted state. Needle gauge may range for example between 34 to 30G and/or **30G** to **26G** and/or **26** to **22G.** The needle retraction stroke is optionally at least 1 mm longer than needle extended length. Optionally the needle may be extended by a spring driven mechanism. For example the force exerted by the spring at the needle may range between 20 to 40 gr (grams force) and between 40 to 80 gr and/or greater than 80 gr.

### DETAILED EMBODIMENTS

Before explaining at least one embodiment of the invention in detail, it is to be understood that the invention is not necessarily limited in its application to the details of construction and the arrangement of the components set forth in the following description and/or illustrated in the drawings and/or the Examples. The invention is capable of other embodiments or of being practiced or carried out in various ways.

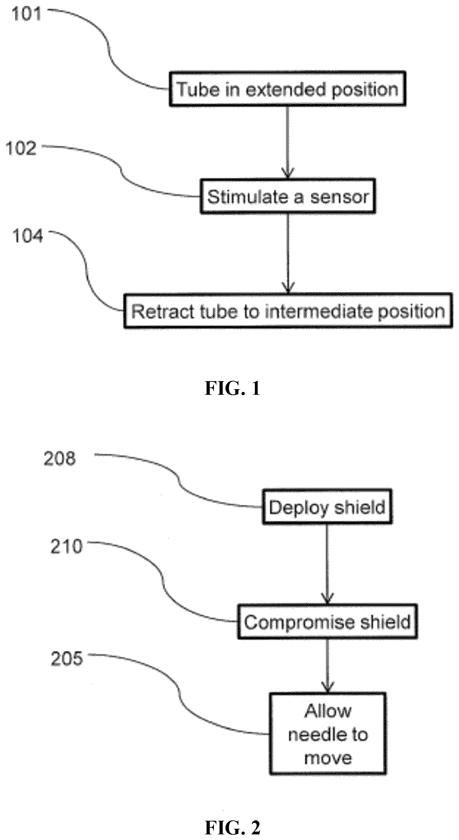

Referring now to the figures, *FIG. 1* is a flow chart illustration of a method of partially protecting a hazardous element, which is not part of the invention, in accordance with an embodiment of the current invention. In some embodiments, a hazardous element includes a sharp hazard and/or a biohazard and/or burn hazard and/or a toxic hazard and/or a chemical hazard (e.g. corrosive). For example a tip of a tube and or a needle may include a sharp hazard. The tube is optionally locked 101 in an extended position. A sensor is optionally mechanically interconnected with a locking mechanism. Stimulating **102** the sensor optionally causes releasing of the lock allowing the needle to be retracted **104** from the extended position to an intermediate position.

In some embodiments a tube (for example a needle and/or a cannula) may be used for discharging a drug into a patient. The tube may be locked **101** in an extended position, for example during discharge.

In some embodiments, drug discharge device will include a sensor. For example a sensor may be stimulated **102** when a device is removed from a patient. For example, a mechanical sensor may be biased to move outward. Outward movement of the sensor may be blocked when the device is on an injection surface, for example on the skin of the subject. When the device is removed from the surface, the sensor may be released and/or move. Alternatively or additionally a sensor sense light and/or may sense heat and/or conductivity of the surface. When the sensor is stimulated **102** for example detecting that the device was removed from the surface, the detector may unlock the tube allowing it to retract **104** from the extended state. Alternatively or additionally, when the sensor detects **102** that the device was removed from the surface, the sensor may trigger retraction of the tube. Alternatively or additionally, when the, when sensor detects that the device was removed from the surface, it may lock a needle retraction mechanism inhibiting the needle from moving to some position. For example, the needle may be inhibited from reaching a fully retracted position. For example the needle may be inhibited from returning to the extended position. Alternatively or additionally, a sensor may include a sensor of a state of the injection device, for example finishing of discharge and/or a malfunction.

*FIG. 2* is a flow chart illustration of a method of neutralizing a hazard, which is not part of the invention, in accordance with an embodiment of the current invention. For example, the hazard may be a stick hazard from a needle point. For example, a system may include a safety backup in case a first neutralizing mechanism is compromised. In some embodiments a needle may be neutralized by shielding **208** the needle. Optionally, the needle may be moved **205** for example to a protected position when the functioning of the shield is compromised **210,** for example by tampering with the shield. For example, compromising a shield may include applying a force to a shield. For example the force may be directed in a failure direction (for example a collapse direction and/or an opening direction). The force may range for example between 10 gr to 100 gr and/or 100 gr to 1000 gr and/or 1000 gr to 5000 gr and/or 5000 gr to 10000 gr. Compromising a shield may include, for example, moving a shield with respect to a hazard and/or a housing. For example, movement of the shield may include, at the point on the shied of maximum movement, movement of between 0.1 mm and 1 mm and/or between 1 mm and 5 mm and/or between 5 mm and 2 cm and/or more than 2 cm.

In some embodiments, allowing the needle to move **205** may include retracting the needle to a protected location. Alternatively or additionally, the needle may be allowed to move and/or fold, for example by unlocking a retraction mechanism and/or a needle holder. For example, once the shield is unlocked, if the shield fails, for example collapses, the shield may cause the needle to collapse and/or be neutralized.

In some embodiments, compromising **210** the shield may include applying a force to the shield. For example when a force is applied pushing the shield towards a collapsed state and/or pulling a shield away from the shielding position, movement **205** of the needle may be triggered. For example when a force is applied to the shield by an object pushing itself between the shield and the needle, movement **205** of the needle may be triggered.

In some embodiments, a shield may be deployed **208** while a needle in an extended position. Optionally a needle may be locked into an extended position while discharging a drug into a subject. For example, after discharge and/or after removal of the discharge device from the subject, a shield may be deployed **208** while the needle remains in the extended position. Alternatively or additionally, shield may be deployed **208** while a needle is in an intermediate position. For example, at the end of discharge and/or when the device is removed, a needle may be partially retracted and/or the shield may be deployed to protect the needle in the partially retracted position.

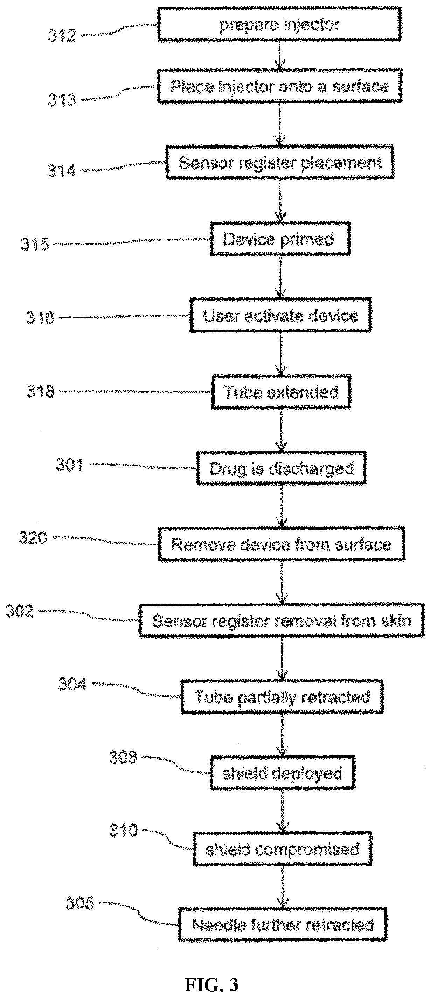

*FIG. 3* is a flow chart illustration of a method of using and/or retracting a tube in accordance with an embodiment of the current invention. In some embodiments, a device may include an automatic tube insertion and/or stick hazard protection system. Optionally, the device may have multiple stages of extension and retraction. For example, an extension mechanism may insert the tube (for example a hypodermic needle) into a subject. For example, a shield may cover the needle and/or ameliorate a stick hazard. For example, a retraction mechanism may move a needle to a fully and/or partially protected position. The shielding and/or retracting are optionally integrated. Optionally, deployment and/or withdrawal of a shield may be triggered by a sensor and/or by a state of the device and/or by a user action. Optionally, extension and/or retraction of a needle may be triggered by a sensor and/or a state of the device and/or a user action. For example, integrated movements of the shield and/or needle may facilitate needle insertion, drug discharges and/or protection against a stick hazard. In some embodiments, a single component may serve multiple functions. For example a sensor may also shield a needle and/or a retraction mechanism may also serve as a sensor. Note that *FIG. 3* illustrates a large number of actions. Some actions of the method, which is not part of the invention, may be performed independently of others. Some of the actions may be performed in the absence of others.

In some embodiments, an injector may be prepared **312** for use. For example, preparing **312** an injector for use may include deploying a skin sensor. Optionally an injector is supplied to a user in a stored state. For example, unwrapping the device (for example removing it from a blister package, removing a protective adhesive covering and/or removing a battery isolator) may prepare **312** the device for use. For example, removing the device from a blister may uncover a sensor and/or activate a mechanical sensor. For example a mechanical skin sensor may be freed to deploy from the base of the device as the device is lifted from a blister package and/or when an adhesive cover is removed from the base.

In some embodiments, an injector may be placed **312** onto an injection surface, for example the skin of a subject. Optionally, a sensor may register **314** placement **313** of the device. For example, a mechanical sensor may register contact with the surface. Alternatively or additionally, the sensor may be operative to register a quality of the placement **313** of the device. For example, a sensor may register **314** the temperature and/or conductivity of the surface and/or a sensor may register **314** the distance from a point on the injection surface to a point on the injector and/or whether a position of the injector with respect to the surface is stable and/or changes. For example, if the distance between the sensor location and the injector surface is not stable, the device may alert the user that the device is improperly placed. Exemplary sensors that might be used and/or have potential to detect proper placement on the skin include for example: capacitance, magnetic and/or optic. Optionally, based on output of one or more sensors, a controller may confirm that a needle shield is closed and/or that the device has established contact with skin.

In some embodiments, a device may be primed **315** before use. For example, priming may be performed automatically when the proper placement **313** of the device is registered. For example, a mechanical sensor may be interconnected with a needle extension mechanism. For example, placement **313** of the device on the skin of a subject may cause movement of the sensor and/or movement of the sensor may unlock the needle extension mechanism. Optionally the priming of the system, for example unlocking of the needle extension mechanism, occurs automatically in response to registering **314** of the sensor. Alternatively or additionally, the priming of the system may require a user action (for example pushing an OK button once the device has been properly placed **313).**

In some embodiments, a device may be activated **316** by a user action. For example, once the device has been placed **313** and/or primed **315,** a user may press a button to trigger needle extension **318.** Optionally the needle extends out of the base of the device and/or into the skin of the subject. In some embodiments, a needle may be hollow and/or a drug may be injected through the needle. Alternatively or additionally, after insertion a needle may be removed leaving behind a tube (for example a cannula) for discharge **301** of a drug. Alternatively or addition, movement of the sensor may automatically trigger activation of the device and/or needle extension **318.** Alternatively or additionally, a user may manually extend **318** the needle (for example the user may push against a surface which drives the needle into his skin). Optionally, a drug may be discharged **301** through the needle into the subject.

In some embodiments, a user may remove **320** a device from a surface. For example after discharge **301** of the drug, a user may remove **320** the device from his skin. Alternatively or additionally, a user may remove **320** the device before discharge has completed (e.g. prematurely). Alternatively or additionally, a user may remove **320** the device when a malfunction occurs. Optionally a sensor may register **302** removal of the device.

In some embodiments, in response to registering **302** removal of the device from the skin, the device may be safeguarded. Alternatively or additionally the device may be safeguarded in response to a completion of discharging and/or a malfunction. Optionally, safeguarding the device includes neutralizing a stick hazard. For example, a delivery tube (e.g. a needle) may be partially retracted **304** and/or a shield may be deployed **308.** For example, in the deployed state, the shield may cover the tip of the needle. For example the shield may deploy **308** to cover the tip of the needle while the needle is in the partially retracted **304** state. In some embodiments, a skin sensor may include a needle shield.

In some embodiments, a safeguard mechanism may include a redundant and/or failsafe protection mechanism. For example, a needle may include a further retraction **305** mechanism that moves the needle to a protected location when there the device detects a possible compromising **310** of the needle shield.

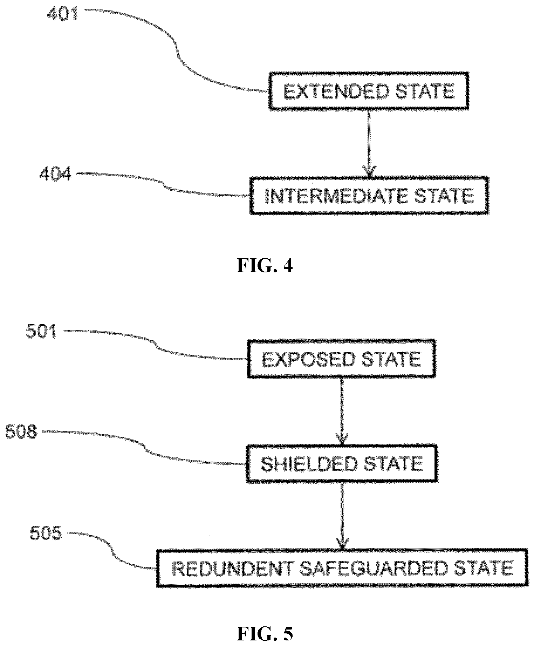

*FIG. 4* is a state diagram illustration of a retractable needle in accordance with an embodiment of the current invention. In some embodiments a drug discharge device may have an active state in which an access tube (for example a hypodermic needle) extends out of the device. Optionally, the device may also have a safeguarded state in which the tube is partially retracted. For example, when discharge of the drug is interrupted in the active state, the device may automatically switch to the safeguarded state.

In some embodiments, in active state **401,** a sharp needle and/or cannula may extend out of the device. In a safeguarded state the needle is optionally partially retracted **404.** For example, in the extended state a portion of the needle may be exposed and/or extend outside a housing of the device. Optionally, in the partially retracted state, a smaller portion of the needle may be exposed and or extend out of the device than in the exposed state. For example, in the extended state a needle tip may extend a distance away from a housing of the device (for example the distance from the device may be operationally defined as the shortest distance between the needle point and any point on the device and/or as the distance between the needle point and a particular location for example a sensor location on the device). Optionally, in the partially retracted state, the needle may extend away from the device less far than in the exposed state. Optionally the device may have a fully retracted state and/or an intermediate state. In the partially retracted state the needle may be on a path between the fully retracted state and the extended state. In some embodiments, a shield may cover a tip of the tube in the partially contracted state.

*FIG. 5* is a state diagram illustration of a needle in accordance with an embodiment of the current invention. In some embodiments, an injector may have an exposed state **501** and/or multiple safeguarded states. For example, in a first safeguarded shielded state **508,** a sharp point of a tube may be covered by shield protecting a user from a hazard. The device may have a second safeguarded state **505.** For example the device may revert to the second safeguarded state when there is a sign of compromise of protection of the first safeguarded state. For example in the second safeguarded state **505** the tube may be retracted to a safe location. For example, when a force is applied to the shield, the tube may be retracted into the second safeguarded state.

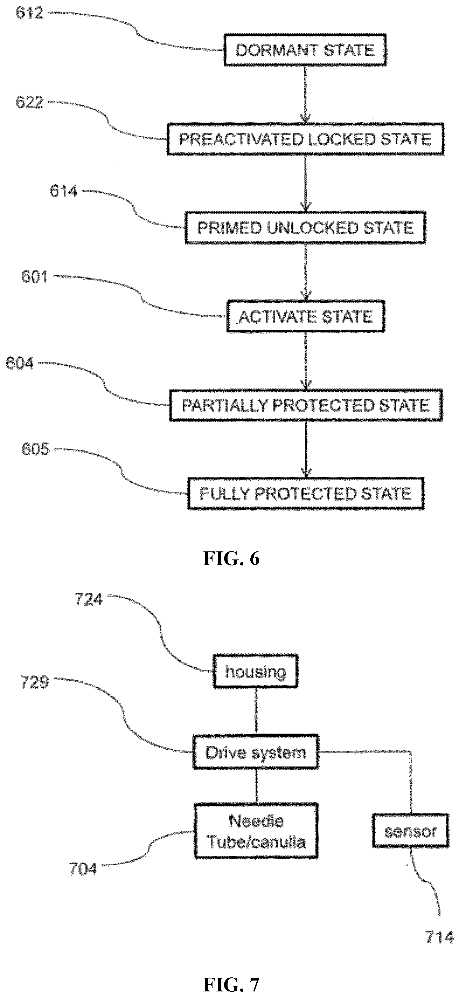

*FIG. 6* is a state diagram illustration of a multi position needle in accordance with an embodiment of the current invention.

In some embodiments an injector may have a dormant **612** state. For example, in dormant state **612** an energy storage device (for example a battery) may be isolated, for example avoiding draining energy. Optionally in the dormant state **612,** a component of the system is positioned to be protected against damage during storage and/or transport. Optionally in the dormant state a hazardous component (for example a needle tip) is locked in a protected position and/or a shield is locked around the hazardous component.

In some embodiments, the device includes a preactivated state **622.** For example the device may enter the preactivated state **622** from a dormant state **612.** For example, the switch to the preactive state may be caused by an action of a user and/or a supplier. For example, the device may enter the preactivated state **622** when the device is removed from a package and/or in response to a user and/or a helper and/or a supplier and/or a medical aid switching the device on. Optionally, in the preactivated state **622** a hazardous component is locked and/or shielded. Optionally in the preactivated state **622** a sensor is initiated.

In some embodiments a device includes a primed state **614.** For example, a device may switch from a preactivated state **622** to a primed state **614** when a sensor registers that the device is ready for use (for example it has been placed on the skin of a subject). Optionally in primed state **614** a hazardous component is unlocked and/or ready for exposure. For example, when a preactivated device is placed on the skin of a user, a skin sensor may register placement on the skin and/or unlock a needle. Alternatively or additionally, a device may not have separate preactivated **622** and primed **614** states. For example, the device may be preactivated and/or primed simultaneously.

In some embodiments, a device may include an activated state **601.** For example, a user may activate a primed device, for example by pressing an activation button. In the active state **601** a hazardous component may be exposed. For example, a needle may be extended out of a housing of the device. For example in the activated state **601** a drug may be discharged through a tube (e.g. an extended hypodermic needle) into a subject.

In some embodiments a device may include a partially protected state **604.** For example at the completion of drug discharge and/or upon a malfunction and/or upon removal of the device from the skin a hazardous component may be partially protected. For example a needle may be shielded and/or partially retracted. The partially protected position is optionally stable. For example, the device may remain in the partially protected state **604** until disturbed.

In some embodiment a device may include a fully protected state **605.** For example, if there is some compromise of the protection of the hazardous component in the partially protected state **604,** the device may revert to a fully protected state **605.** For example, if a force is applied to a needle shield protecting a needle in a partially protected state **604,** the needle may retract to a fully protected state **605.** Optionally a device may be permanently locked and/or disabled in one or both of the partially protected state **604** and/or the fully protected state **604.**

*FIG. 7* is a block diagram illustration of a drug delivery system in accordance with an embodiment of the current invention. Optionally, a device may include a drive system **729.** Optionally, drive system **729** may expose and/or protect a hazardous component. For example a hazardous component may include a sharp needle **704.** For example drive system **729** may expose a sharp needle by extending it out of a housing **724.** For example a drive system **729** may protect a sharp needle **704** by retracting it back into housing **724.** In some embodiments there may be an intermediate state between a fully retraction and extension (for example a partially retracted state). For example drive system **729** may move a hazardous component to an intermediate position and hold it there. Optionally, drive system **729** is responsive to a sensor **714.** For example, drive system **729** may be connected to sensor **714** by means of an electromechanical and/or a mechanical connection and/or through a logical component (for example a processor). In some embodiments, a drive system and/or a sensor may include an electro magnet, and/or a linear actuator and/or a piezoelectric element.

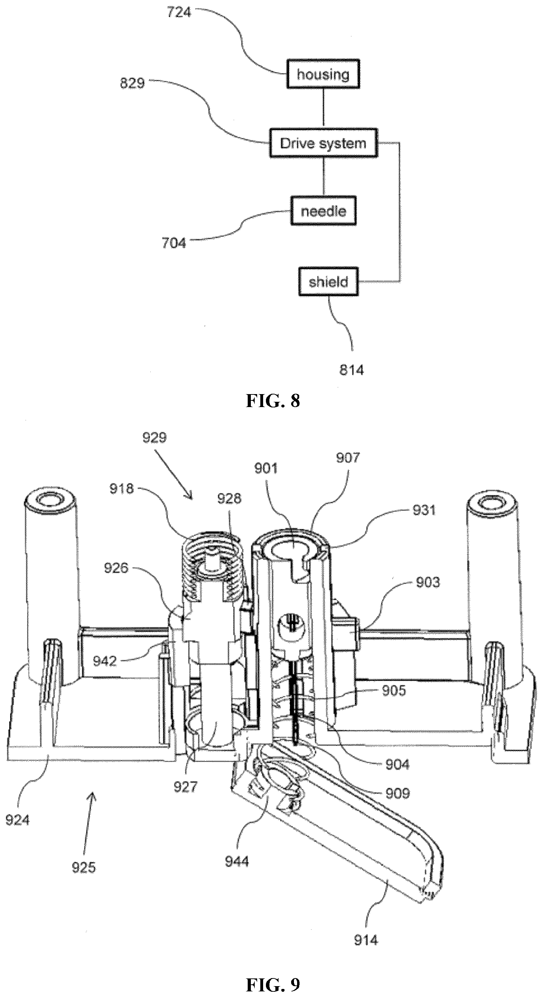

*FIG. 8* is a block diagram illustration of a protection mechanism in accordance with an embodiment of the current invention. Optionally a protection mechanism may include a shield **814.** For example, in some configurations shield **814** may cover and/or protect a hazardous element (e.g. a needle **704).** Optionally shield **814** may be synchronized with a drive system **829.** For example, needle **704** may be protected by shield **814** when shield **814** is in a deployed position and needle **704** is in a retracted position and/or an intermediate position. For example, needle **704** may retract when shield **814** deploys. For example, needle **704** may partially retract when shield **814** deploys. Optionally, needle **704** is retracted into a housing **724** needle **704** and/or protected when shield **814** is withdrawn and/or compromised. Optionally when shield **814** is withdrawn and/or compromised, needle **704** may retract to inside a housing **724.** For example, a housing may surround, partially surround and/or protect an inner space. Optionally or additionally a medicine to be injected may be stored on the inside of an injector.

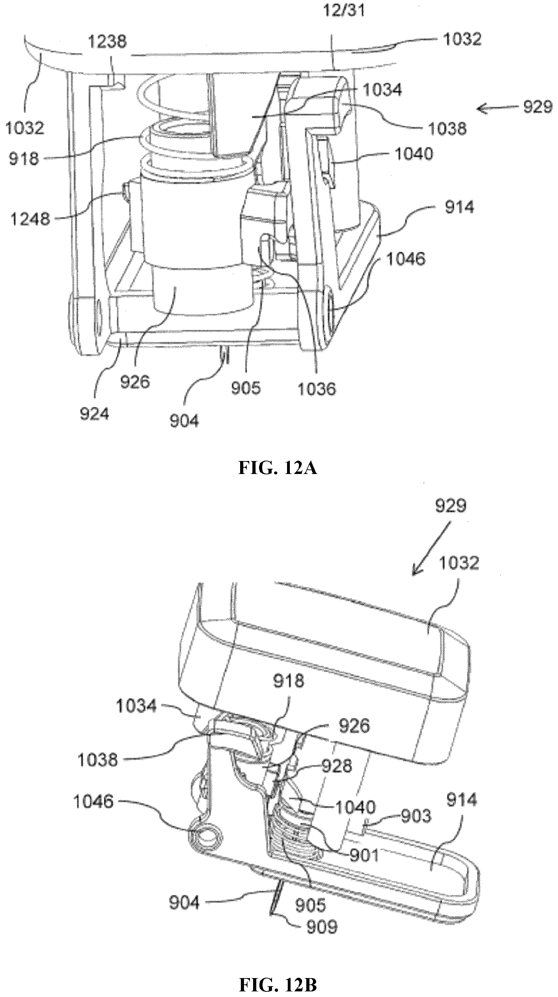

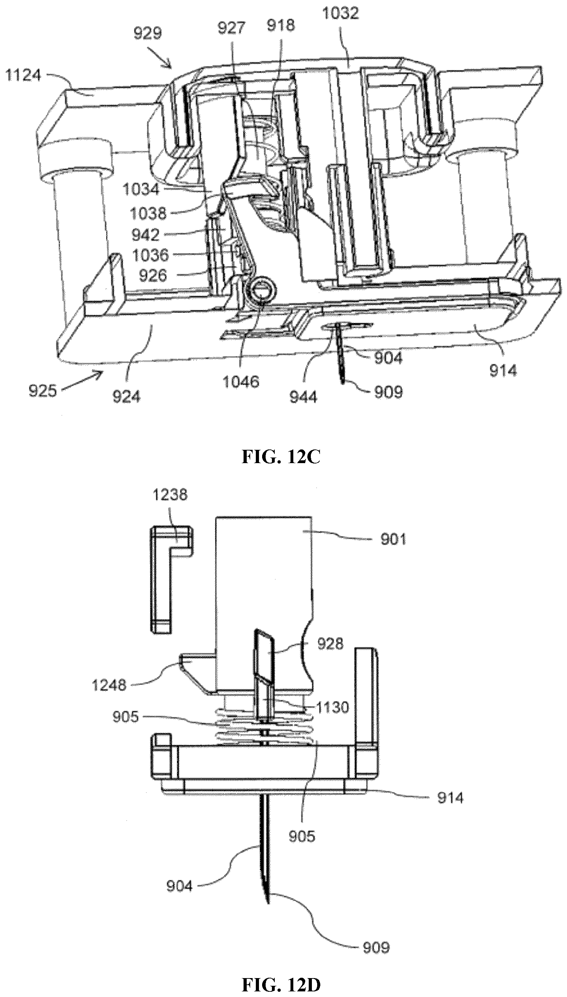

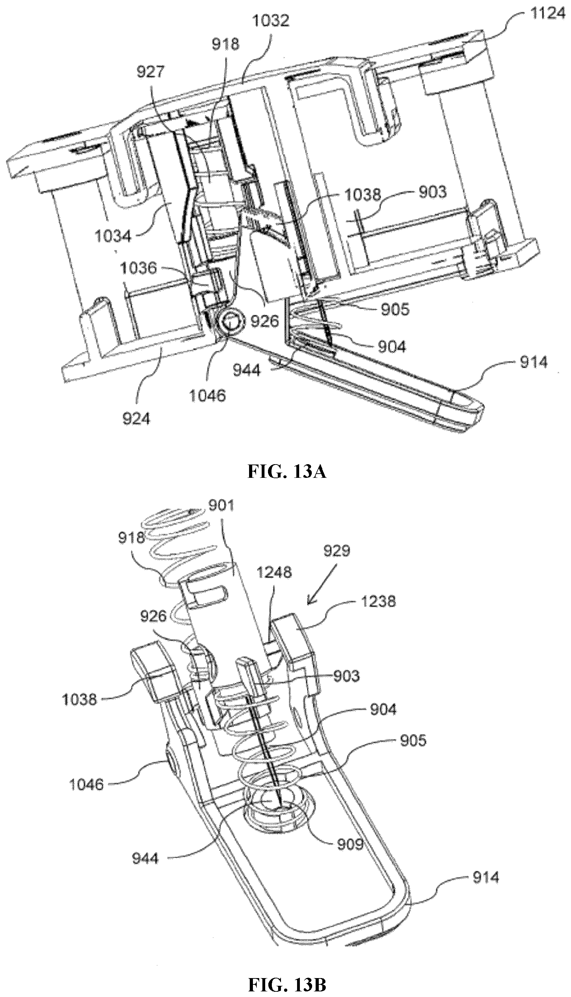

*FIG. 9* is a perspective cutaway cross sectional illustration of a multistate needle insertion and protection system in accordance with an embodiment of the current invention. The system includes a tube (e.g. a hypodermic needle **904** with a tip **909** that is optionally sharpened) mounted on a needle retainer **901.** Retainer **901** optionally moves along a path. For example the path may be defined by a cylindrical track **907** between a retracted position (for example as illustrated in *FIG. 9**-**FIG. 11C*) and/or an extended position (for example as illustrated in *FIGs. 12A-12D*) optionally including an intermediate, partially retracted position (for example, as illustrated in *FIGs 13A-13C*)*.*

In some embodiments a needle may be connected to a fluid train. For example, a needle may have a sharp point and/or a lumen. An opening to the lumen may be connected to the fluid train. For example, the fluid train may supply a connection between the lumen of a needle and a medicine cartridge. For example, the fluid train may be connected to and/or be in fluid communication with the lumen. For example, the fluid train may be connected to the needle or tube on an end opposite a sharpened end and/or opposite an end that is configured for contacting a subject and/or penetrating a skin of a subject.

In some embodiments, in the retracted position, needle **904** is located in a protected position. For example, in *FIG. 9* a sharp tip of needle **904** is pulled back behind a contact surface **925** of base **924** of the device. Optionally, in the retracted position the entire needle **904** and/or a sharp tip thereof is surrounded by a housing. For example, in *FIG. 9*, needle **904** is surrounded by track **907.**

In some embodiments a shield **914** is provided. Shield **914** optionally moves between a deployed position and a withdrawn position. An exemplary deployed position is illustrated in *FIG. 9* wherein a portion of shield **914** is extended outward from contact surface **925.** Optionally in the deployed position, a needle aperture **944** is disaligned with needle **904,** for example, inhibiting extension of needle **904** past shield **914** (for example as illustrated in *FIG. 13A*). Optionally when shield **914** is in a withdrawn position aperture **944** is aligned with needle **904,** for example allowing extension of needle **904** (for example as illustrated in *FIG. 12C*).

In some embodiments, a drive system **929** controls movement of needle **904.** Optionally, drive system **929** includes an insertion mechanism. For example an insertion mechanism may cause a discharge tube (e.g. needle **904)** to move outward and/or to protrude outward from skin contact surface **925** and/or to penetrate the skin of a subject. Alternatively or additionally drive system **929** includes a retraction mechanism that returns a discharge tube backwards from a protruding position towards a position behind contact surface **925.** For example, in the embodiment of *FIG. 9* a drive system **929** includes both an insertion and a retraction mechanism.

In some embodiments, drive system **929** includes a path defining element. For example a track and/or a carriage running along the track and/or a hinge and/or an axle and/or a selective movement blocking element. For example drive system **929** include track **907** and a carriage that runs along the track, for example a carriage may include a needle retainer **901.** Optionally, needle retainer **901** slides along an inner bore of track **907** defining a linear path of movement of needle **904.** For example in system **929,** track **907** has a circular bore. Optionally, rotation alignment of retainer **901** is controlled via a tab **903** of retainer **901** moving along a slit **931** in track **907.** Optionally slit **931** is linear to keep retainer **901** at a fixed rotational orientation. Alternatively or additionally, a slit may include a curve and/or a selective blocking elbow to cause rotation of a carriage and/or selectively block movement of a carriage dependent on its rotation orientation. Optionally slit **931** is blocked at a bottom point to limit movement of needle retainer **901** and/or to limit protrusion of needle **904.** Optionally, tab **903** may include an indicator of needle position. For example an optical sensor may be used to track the position of tab **903** and/or needle **904.**

In some embodiments, drive system **929** includes a second track **927** and carriage (e.g. driver **926).** For example, driver **926** slides over track **927** along a linear path of movement. For example, selective movement blocking element **942** selectively blocks downward movement of driver **926** dependent on the rotational orientation of driver **926.** Optionally, a track may define a linear and/or a curved path of movement. In some embodiments, a needle may have a linear and/or curved path of movement. Alternatively or additionally, a needle may move on an arm mounted on a pivot and/or another path determining mechanism. Alternatively or additionally, locking and/or facilitating and/or limiting of movement may be controlled by mechanisms other than rotational alignment. For example, a needle and/or needle mount and/or needle driver and/or needle retainer may include a locking mechanism with an elastic element and/or a lock (for example a barb and/or an interference element).

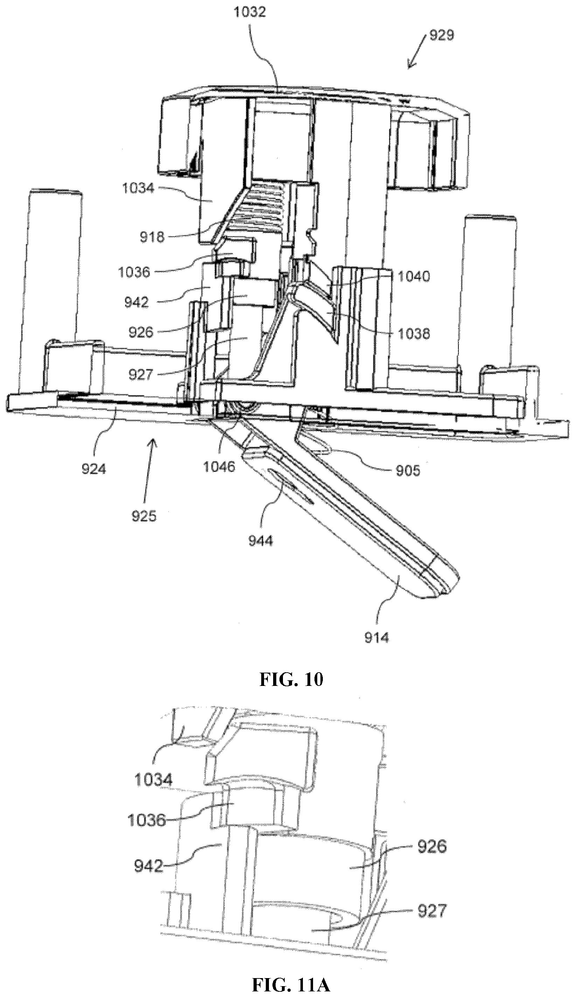

Optionally, drive system **929** includes one or more energy storage device and/or actuators and/or forcing elements. For example, drive system **929** includes three forcing elements: a forced biased insertion spring **918,** a backward biased shield actuator and/or retraction spring **905** and a manual actuator (for example a manual actuator may include a push button **1032** as illustrated in *FIG. 10*). Alternatively or additionally, an actuator and/or energy storage device may include an elastic element and/or an electrical actuator and/or a battery and/or a pressure reservoir and/or a pneumatic actuator and/or a hydraulic actuator and/or a chemical reservoir and/or a chemically reaction driven actuator.

In some embodiments, a drive system includes one or more control elements. Optionally, control elements may include interconnected moving elements that block and/or trigger movement of a needle and/or a needle shield and/or an actuator. A control element is optionally interconnected to and/or responsive to a sensor. For example, control elements may include a gear and/or a wheel and/or a friction element and/or an interference element and/or a flange and/or a rotating element and/or a sliding element. Alternatively or additionally, control elements may include a switching element and/or a logical processor. For example, a sensor may include a mechanical sensor interlocked with a mechanical control element. Alternatively or additionally, a sensor may include a mechanical sensor interlocked with a logical control element, for example a transducer and/or a processor. Alternatively or additionally a sensor may include other forms of sensors and/or transducers for example an optical sensor and/or a pressure transducer and/or a heat transducer. Interconnections between control elements may include mechanical connections, wired electrical connections, flow paths, and/or wireless connections.

In some embodiments, drive system **929** includes interconnected control elements. Optionally the drive elements are mechanically interlocked. For example, mechanical control elements of drive system **929** include a driver **926.** For example, driver **926** may be actuated by spring **918** and/or include an interference element **928.** For example, interference element **928** interconnects between needle driver **926** and needle retainer **901.** In the exemplary embodiment, mechanical control elements of drive system **929** may be connected to and/or responsive to a mechanical skin sensor. For example, a skin sensor may include a shield **914.** Optionally, shield **914** is deployed outward from surface **925** by spring **905.** Optionally, shield **914** is interconnected to needle retainer **901** and/or button **1032** and/or driver **926.** For example, mechanical control elements of drive system **929** including needle retainer **901** which is optionally biased inward from surface **925** by spring **905** may be interconnected to shield **914.**

*FIG. 10* is a perspective cutaway cross sectional illustration of a mechanism holding a needle in a preactivated state in accordance with an embodiment of the current invention. In some embodiments, in the preactivated state a sensor is activated and/or waiting to sense placement on a surface. For example, needle shield **914** may include a mechanical sensor that is deployed outward from surface **925** (for example as illustrated in *FIG. 10*). Optionally, in the preactivated state, activation button **1032** and/or needle **904** and/or driver **926** are locked in an inactivated and/or retracted state. For example, locking may inhibit premature extension of needle **904.**

In some embodiments, a skin sensor element (e.g. shield 914) is interconnected to a control element and/or an actuator of driver system 929. For example interlocking may inhibit premature needle extension. For example, shield 914 interconnects to button 1032. For example, an interference element (e.g. a flange 1038) is integrated to shield 914. When shield 914 is in the deployed position, flange 1038 optionally blocks downward movement of a corresponding interference element (e.g. protrusion 1040) integrated to button 1032. For example, depression of button 1032 and/or activation of the device may be inhibited until the sensor detects placement on a surface.

In some embodiments, in the preactivated state, a control element is interlocked to a path defining element. For example, an interference element of driver 926 (e.g. protrusion 1036) may be interlocked to a selective blocking element **942.** For example, the interlocking may inhibit downward movement of driver **926** and/or inhibit driver **926** from pushing needle retainer **901** and/or needle **904** outward. An exemplary 3-D form of protrusion **1036** is illustrated in more detail in *FIGs. 11A* *and* *12A**.* Button **1032** optionally includes a transmission element (e.g. wedge **1034)** that transforms a force in a first direction on button **1032** to a force in a second direction on driver **926.** For example, a downward force on button **1032** is transformed to a sideways force on protrusion **1036** and/or a torque on driver **926.**

In some embodiments shield **914** rotates around a pivot **1046** for deployment away from surface **925** and/or withdrawal towards surface **925.** In some embodiments, shield **914** includes an aperture. When shield **914** is deployed aperture **944** is optionally misaligned with needle **904** such that the point of needle **904** is blocked by shield **914.**

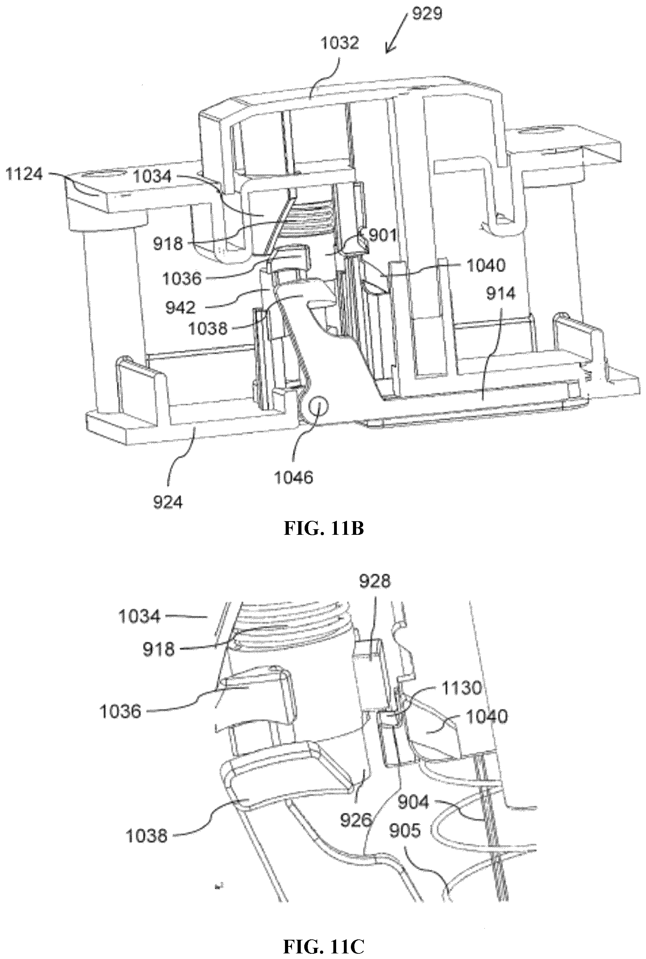

*FIGs. 11A-11C* are a perspective cutaway and cross sectional illustrations of a needle mechanism in a primed state in accordance with an embodiment of the current invention. In some embodiments, placing a preactivated drug delivery device against a surface primes the device. For example, placing contact surface **925** against the skin causes a skin sensor to register the skin and/or prime the device. For example, in the preactivation state, when surface **925** is placed against a surface, shield **914** is collapsed inward towards surface **925** from a deployed state optionally compressing spring **905** (for example as illustrated in *FIG. 10*). For example in *FIG. 11B**,* shield **914** is illustrated collapsed. In some embodiment, shield **914** moves by rotation **1046** approximately 10 degrees around axis. Optionally in the collapsed state the outer surface of shield **914** is approximately flush with surface **925.** For example, collapse of shield **914** causes flange **1038** to move out of the way of protrusion **1040** allowing button **1032** to be depressed. Optionally, depressing button **1032** releases a control element automatically activating the device. In some embodiments, rotation of a shield may range between 0 to 10 degrees and/or between 10 to 30 degrees and/or between 30 to 60 degrees and/or between 60 to 90 degrees and/or between 90 to 120 degrees and/or between 120 to 180 degrees.

In some embodiments, a transmission element of an actuator is aligned with an interference element of a control element. For example, in a primed state, wedge **1034** of button **1032** is aligned with protrusion **1036,** for example as illustrated in *FIG. 11A**.* Optionally, depressing button **1032** will push wedge **1034** against protrusion **1036.** As wedge **1034** is pushed against protrusion **1036,** it optionally transmits a lateral force onto protrusion **1036.** For example, the lateral force may cause a torque on driver **926** (for example due to the force couple between the driving force of wedge **1034** on protrusion **1036** and the counter force of track **927).** The torque will optionally rotate driver **926** around track **927.** Rotating driver **926** optionally disengages protrusion **1036** from blocking element **942,** for example as illustrated in *FIGs. 11A-**11C.*

In some embodiments, rotating drive **926** aligns an interference element **928** with a corresponding interference element **1130** on needle retainer **901,** for example as illustrated in *FIG. 11C**.*

In some embodiments, a drug discharge device includes an upper housing. For example, a portion of an upper housing **1124** is illustrated in *FIG. 11B**.* Optionally a needle driver system may be completely surrounded by base **924** and/or upper housing **1124.**

*FIGs. 12A-12D* are perspective cutaway and cross sectional illustrations of a needle mechanism in an activated and/or extended state in accordance with an embodiment of the current invention. In some embodiments, a user activates a device from a primed state to an active state. For example, by a user depressing button **1032,** driver **926** is rotated, aligning interference element **928** of driver **926** with interference element **1130** of needle retainer **901** and/or disengaging protrusion **1036** from selective blocking element **942.** When protrusion **1036** is disengaged from selective blocking element **942,** spring **918** pushes driver **926** downward. As driver **926** moves downward interference element **928** optionally engages interference element **1130** and/or drives needle retainer **901** downward and/or extends needle **904** outward. Optionally driver **926** is held down by spring **918.** Optionally, while shield **914** is in the withdrawn position and driver **926** is in the extended position, driver **926** is prevented from disengaging from needle retainer **901.**

In some embodiments, in the active stage needle **904** is locked in the extended position. For example, needle **904** may extend into the skin of a subject and/or act as a fluid path for discharging the drug into the subject. Optionally, needle **904** may be locked in the extended position by driver **926.** For example, interference element **928** may remain engaged to interference element **1130** preventing upward movement of needle retainer **901** and/or retraction of needle **904.** For example, while shield **914** is in the withdrawn position, shield **914** engages protrusion **1036** and/or prevents driver **926** from rotating. As long as driver **926** is prevented from rotating, interference element **928** of driver **926** optionally remains engaged with interference element **1130** of needle retainer **901,** for example as illustrated in *FIG. 12A**.*

In some embodiments, while shield **914** is in the withdrawn position, aperture **944** is aligned with needle **904** allowing needle **904** to extend out aperture **944** as is illustrated for example in *FIGs. 12B -12C**.*

*FIG 12A* illustrates an optional interference element (e.g. a flange **1248)** on needle retainer **901.** Optionally, needle shield **914** includes an interference element (e.g. a lug **1238).** For example, when shield **914** is in a withdrawn position (for example as illustrated in *FIG. 12C*) lug **1238** is not aligned with flange **1248.** For example, when shield **914** is in a deployed position (for example as illustrated in *FIG. 13A*) lug **1238** is aligned with flange **1248.**

*FIGs. 13A-13C* are perspective cutaway illustrations of a mechanism holding a needle in an intermediate state in accordance with an embodiment of the current invention. In some embodiments, when the base **924** is removed from an injection surface (e.g. the skin of a subject), shield **914** moves outward to the deployed position. For example shield **914** pivots outward around pivot **1046.** Optionally outward movement of shield **914** facilitates retraction of needle **904.** Additionally or alternatively, needle may partially retract and/or be held in a stable partially retracted position.

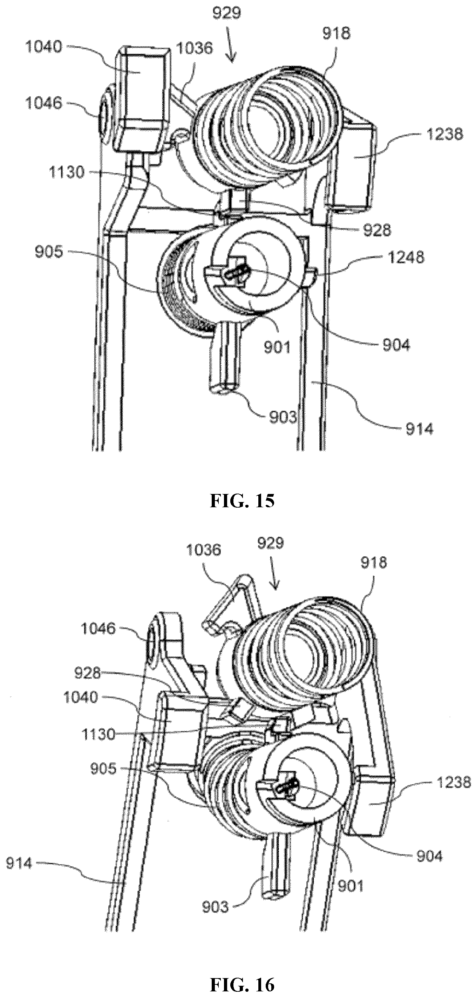

In some embodiments, in the active state, driver **926** is biased to disengage from needle retainer **901.** For example, in the active state driver **926** is biased to rotate by the force of spring **905** against the angled interface between interference element **928** and interference element **1130.** In the active state disengagement is optionally blocked by interference between shield **914** and protrusion **1036,** for example as explained in regards to *FIG. 12A* and as illustrated in *FIG. 15**.*

In some embodiments, deploying of shield **914** from a withdrawn state in an active state to a deployed state (for example by removing the injector from the skin of a subject) unlocks needle **904** and/or or facilitates needle retraction. Optionally, deploying shield **914** from the active/withdrawn state frees driver **926** to rotate. For example, as illustrated in *FIGs. 12A* *and* *15**,* in the active state, shield **914** interferes with movement of protrusion **1036** and/or inhibits rotation of driver **926.** Optionally, as can be seen for example in *FIGs. 13A* *and* *16**,* when shield **914** is deployed, the body of shield **914** is distanced from protrusion **1036** enough to not interfere with rotation and/or to allow rotation of protrusion **1036** and/or driver **926.**

Optionally, when driver **926** rotates from the active position (for example of *FIG. 15*) to the partial retracted position (for example of *FIG. 16*) interference elements **928** and **1130** disengage. Disengaging interferences element **928** allows interference element **1130** and/or needle retainer **901** and/or needle **904** to move inward (e.g. retract).

In some embodiments, while shield **914,** is in the deployed position, needle **904** retracts to and/or is held in the partially retracted position. For example as illustrated in *FIG. 13B**,* shield **914** may be integrated to an interference element or flange (e.g. lug **1238).** When shield **914** is deployed, interference lug **1238** optionally blocks a retraction path of needle **904.** For example, lug **1238** blocks retraction of needle **904** before it reaches a fully retracted position (for example before it reaches the fully retracted state illustrated in *FIGs. 9-11C*). For example, as needle **904** retracts flange **1248** contacts lug **1238** stopping retraction in the partially retracted state. Optionally, while shield **914** remains in the deployed state, needle **904** is held stable in the partially retracted state.

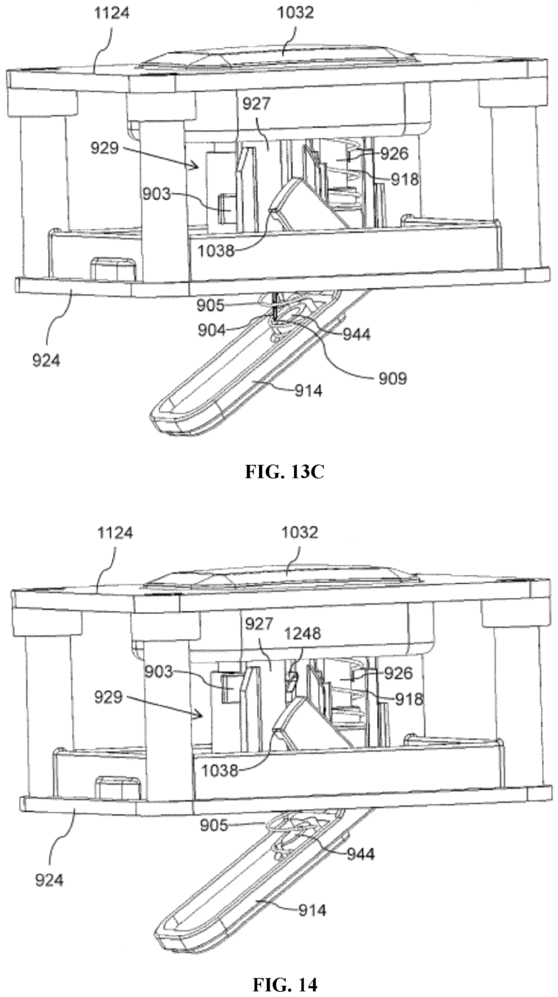

In some embodiments, in the deployed position, shield **914** covers a tip of needle **904.** For example, in the deployed position aperture **944** may be misaligned with needle **904** (for example as illustrated in *FIGs. 13A* *and* *13C*). For example, in the extended and/or partially retracted position, needle **904** may block shield **914** from withdrawing from the deployed position to the withdrawn position. Optionally, shield **914** may be positioned very close to the tip of needle **904.** For example, close positioning of shield **914** over needle **904** may inhibit insertion of a finger between shield **914** and needle **904** may inhibit a stick hazard.

Optionally, for example as illustrated *FIG. 13C**,* in the intermediate state a portion of needle **904** may extend out of base **924.** For example, in the intermediate state needle **904** may protrude from base **924** less than in the active state. Optionally, in the intermediate state the needle may be protected, for example by shield **914.**

*FIG. 14* is a perspective cutaway illustration of a mechanism holding a needle in a retracted state in accordance with an embodiment of the current invention. In some embodiments, disturbing shield **914** in the deployed position may cause needle **904** to collapse from an intermediate position to a further and/or fully retracted position. For example when shield **914** moves, flange **1038** may disengage from flange **1248.** Disengagement of flange **1038** from flange **1248** may allow needle **904** to retract. Optionally, retraction may be driven by an actuator, for example spring **905.** Alternatively or additionally, needle **904** may be freed to retract such that collapse of shield **914** from the deployed to the withdrawn position may push needle **904** and or further retract the needle. For example, shield **914** is pushed towards base **924** needle **904** may retract to a protected position inside of housing **1124.**

*FIG. 15* is a perspective cutaway illustration of a needle mechanism in an activated state in accordance with an embodiment of the current invention. Optionally in the activated state, needle **904** is held in an extended state by needle holder **901.** For example, needle holder **901** is held in the extended position by contact between an interference elements on driver **926** (e.g. interference element **928)** and retainer **901** (e.g. interference element **1130).** In turn, driver **926** is optionally held extended towards the base of the device by an actuator (e.g. spring **918).** Interference element **928** optionally is kept engaged with interference element **1130** by interference between a protrusion **1036** on driver **926** and shield **914** (e.g. protrusion **1040** and the portion of shield **914** to which it is attached).

*FIG. 16* is a perspective cutaway illustration of a mechanism holding a needle in an intermediate state in accordance with an embodiment of the current invention. In some embodiments, when the device is removed from a surface, shield **914** pivots outward. Movement of shield **914** optionally allows further retraction of needle **904.** For example, pivoting of shield **914** ends interference between protrusion **1036** and the extension of shield **914.** Without interference between protrusion **1036** and shield **914,** driver **926** optionally rotates. For example, as illustrated in *FIG. 16**,* rotation of driver **926** disaligns interference element **928** from interference element **1130** optionally allowing interference element **1130** and/or needle retainer **901** and/or needle **904** to retract. Optionally, needle retraction is stopped at an intermediate position for example as described above with respect to *FIG. 13C**,* by interference between flange **1248** of needle retainer **901** and lug **1238** of shield **914.**

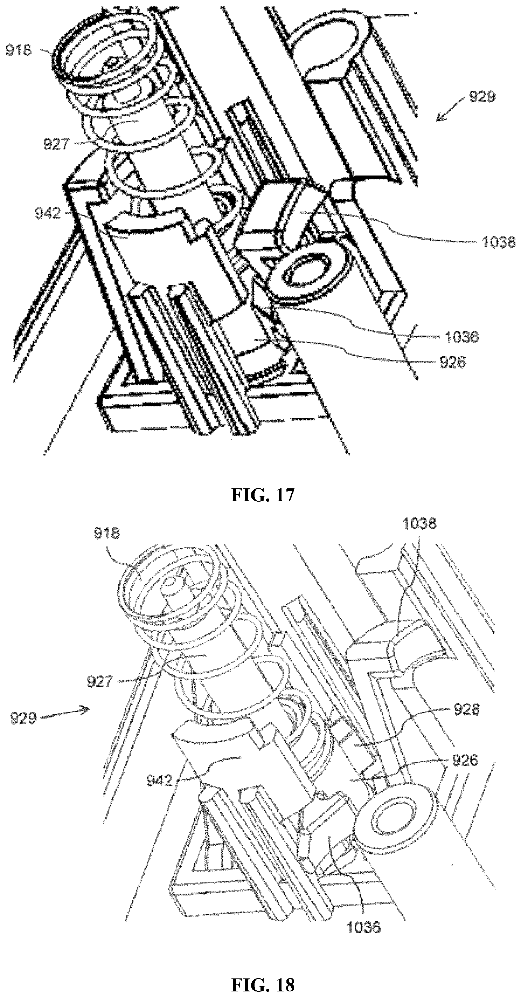

*FIG. 17* is a perspective cutaway illustration of a mechanism holding a needle in an extended state in accordance with an embodiment of the current invention.

*FIG. 18* is a perspective cutaway illustration of a mechanism holding a needle in an intermediate state in accordance with an embodiment of the current invention.

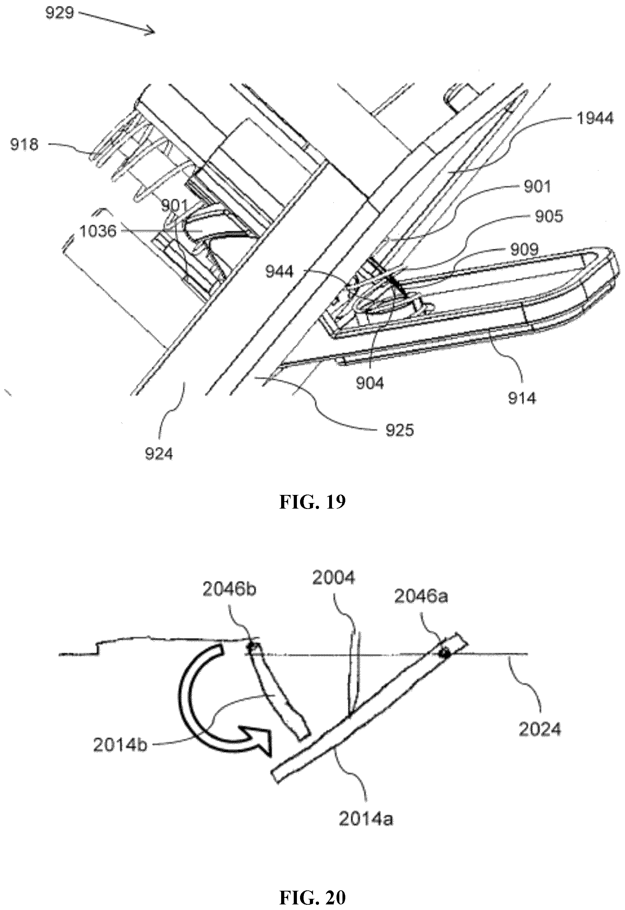

*FIG. 19* is a perspective cutaway illustration of a mechanism holding a needle in an intermediate state in accordance with an embodiment of the current invention. In some embodiments needle shield **914** may include an aperture **944.** Optionally when shield **914** is deployed, aperture **944** is disaligned with needle **904.** For example, outward movement of needle **904** is blocked by shield **914** in the deployed position. Alternatively or additionally, in the partially retracted position, needle **904** may be locked, inhibiting outward movement.

In some embodiments, base **924** includes an opening **1944.** Optionally in the withdrawn position, needle shield **914** covers opening **1944.** For example in the withdrawn position, the outer surface of shield **914** may be approximately flush with surface **925.** Alternatively or additionally, a base may have an indentation and/or shield **914** in the withdrawn position may fit into an indentation that does not penetrate through the base.

*FIG. 20* is a schematic illustration of dual needle shields in accordance with an embodiment of the current invention. In some embodiments, a second needle shield may be used to fortify a first shield against movement due to external forces and/or to block access to a stick hazard. For example, a primary shield **2014a** may cover a stick hazard. A secondary shield **2014b** may prop up primary shield **2014a** and/or block an opening between shield **2014a** and a base **2024** of the device.

In some embodiments, a primary shield may rotate around a first axis **2046a.** Optionally a secondary shield may rotate around a second access **2046b.** For example, shield **2014a** may lie in a withdrawn state over needle **2004** in a retracted state and may open by movement to an acute angle (e.g. counter clockwise in *FIG. 20*) overlying needle **2004.** Alternatively or addition, shield **2014a** may have a withdrawn position flat against base **2024,** but not overlying the needle and/or may open (e.g. clockwise in *FIG. 20*) to an obtuse angle overlying needle **2004.** For example, shield **2014b** may lie in a withdrawn state not overlying needle **2004** in a retracted state. For example needle shield **2014b** may open to an obtuse angle (e.g. counter clockwise in *FIG. 20*). Alternatively or addition, shield **2014b** may have a withdrawn position overlying the needle and/or may open (e.g. clockwise in *FIG. 20*) to an acute angle.

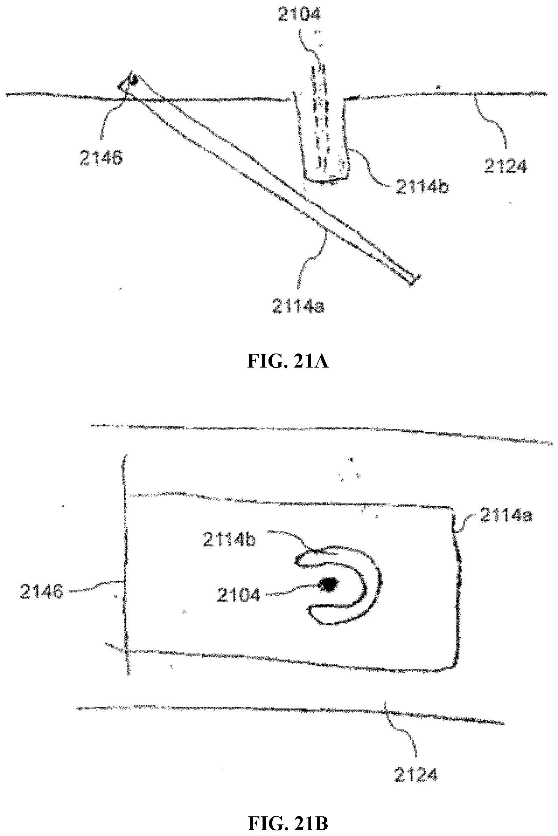

*FIGs. 21A and 21B* are schematic illustrations of needle protection mechanism including a translating needle shields in accordance with an embodiment of the current invention. In some embodiment, a needle shield may include a translating part. For example a sleeve **2114b** may deploy by translating parallel to the axis of a needle. For example, sleeve **2114b** may deploy by translating outward from a base **2124** to cover a needle. For example, shield **2114b** deploy at the end of delivery of a drug and/or when the device is removed from the subject. Optionally sleeve **2114b** may partially surround needle **2104.** For example, as shown in *FIG. 21B**,* shield **2114b** surrounds needle **2104** on three sides. Alternatively or additionally, a sleeve may entirely surround a needle and/or shield a needle from one side. In some embodiments shield **2114b** may lock open. Alternatively or additionally, shield **2114b** may lock to needle **2104.** For example when shield **2114b** is locked to needle **2104,** moving shield **2114b** may also move needle **2104.** For example, forcing shield **2114b** inward (e.g. towards its withdrawn position) may also force needle **2104** inwards and/or retract needle **2104.** Alternatively or additionally compromising shield **2114b** (e.g. applying a force to shield **2114b)** may trigger automatic retraction of needle **2104.** In some embodiments a system with a translating shield **2114b** may also include a pivoting shield **2114a,** for example pivoting around a pivot **2146.** Alternatively or additionally, a translating shield **2114b** shields a needle without a pivoting shield. For example, shield **2114b** may be pushed downward by a spring and/or pulled down by shield **2114a.**

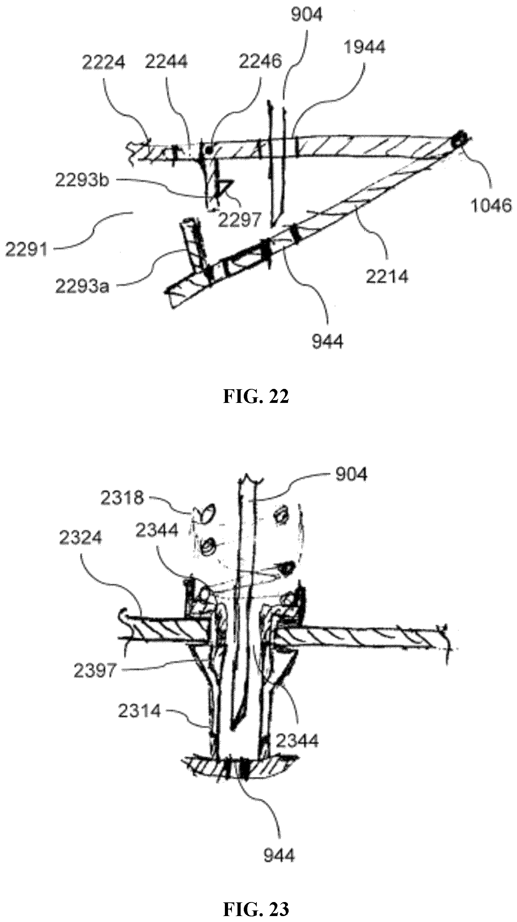

*FIG. 22* is an illustration of a needle point protection device with a protrusion blocking a path to a needle point in accordance with an embodiment of the current invention. In some embodiments, a protection device may leave a path of access to a sharp hazard. For example, a needle shield **2214** may deploy shielding a needle point (for example such that the needle point is located between shield **2214** and a housing of the device (for example a base **2224)).** In some embodiments a gap **2291** provides a path of access to the needle point and/or a path for potential exposure to the hazard. Optionally, a protrusion (for example protrusions **2293a** and/or **2293b)** blocks gap **2291.** For example, protrusions **2293a** and/or **2293b** may be deployed and/or withdrawn.

In some embodiments, a protrusion may be deployed with a needle shield. For example, protrusion **2293a** is integral to and/or connected to shield **2214** such that deploying shield **2214** to cover a tip of needle **904** automatically deploys protrusion **2293a** to at least partially block gap **2291.** Optionally a channel **2244** may be provided into which protrusion **2293a** fits when it is withdrawn. For example, when shield **2214** is withdrawn, protrusion **2293a** fits into channel **2244** in base **2224.** Optionally, withdrawing shield **2214** and/or protrusion **2293a** avoids interference by the shield with the functioning of the device. For example, when the device is active, shield **2214** is flush with base **2224** and/or protrusion **2293a** is inserted into channel **2244** such that in the withdrawn state shield **2214** and/or protrusion **2293a** do not interfere with contact between the outer side of base **2224** and the skin of the subject.

In some embodiments a path to a hazardous component may be blocked by a protrusion extending from a housing of a device. For example, protrusion **2293b** at least partially blocks gap **2291.** Optionally, multiple protrusions **2293a** and **2293b** may cooperate and/or intermesh to block gap **2291.** In some embodiments, protrusion **2293b** may be withdrawable. For example, when a device is placed on the skin of a user and/or in an active mode, protrusion **2293b** may withdraw into base **2224,** facilitating contact between base **2224** and the skin of a user. Optionally, when the device is removed protrusion **2293a** deploys.

In some embodiments, a protrusion may include a sensor. Optionally, protrusion **2293b** may sense a distance between base **2224** and a skin of a user (for example the distance may be the shortest distance from the base to a contact point on the sensor for example for shield **914** the distance to the skin may be operationally measured as the distance from the distance from the far end of shield **914** [opposite pivot 1046] to base **924** along a line perpendicular to the plane of base **924).** For example, protrusion may be biased outward and/or be pushed inward by skin as base **2224** is placed on the skin. Optionally, a sensor (for example a linear gauge sensor) measures the distance that protrusion **2293b** protrudes from base **2224.** That distance may be a measure of the distance from a skin of the user. Optionally, protrusion withdraws when the device is placed straight onto skin, but locks when a force is applied towards needle **904.** For example, if a user tries to push his finger in to gap **2291** towards needle **904,** he will push protrusion **2293b** rightward. The rightward force optionally locks protrusion **2293b** in the protruding state. For example, the protrusion **2293b** may be locked in the protruding state by a ratchet **2297** and/or a pivot **2246.** For example, pivot **2246** may be biased clockwise, disengaging ratchet **2297** unless a significant force is applied rightward. Alternatively or additionally, pivot **2246** may allow protrusion **2293b** to collapse in one direction (for example clockwise) which is not associated with a hazard (for example a finger penetrating gap **2291)** and/or not in a second direction (for example counter clockwise) that is associated with a finger penetrating gap **2291.**

*FIG. 23* is a schematic illustration of a needle protection device with a linear deploying needle shield in accordance with an embodiment of the present invention. Optionally a shield **2314** includes a tubular body and a locking mechanism (for example barbs **2397).** Optionally, at the end of injection and/or when the device is removed from the skin of a recipient, an actuator (for example spring **2318)** pushes shield **2314** out through needle hole **2344** in base **2324.** For example, barbs **2397** lock shield **2314** in an extended position shielding needle **904.**

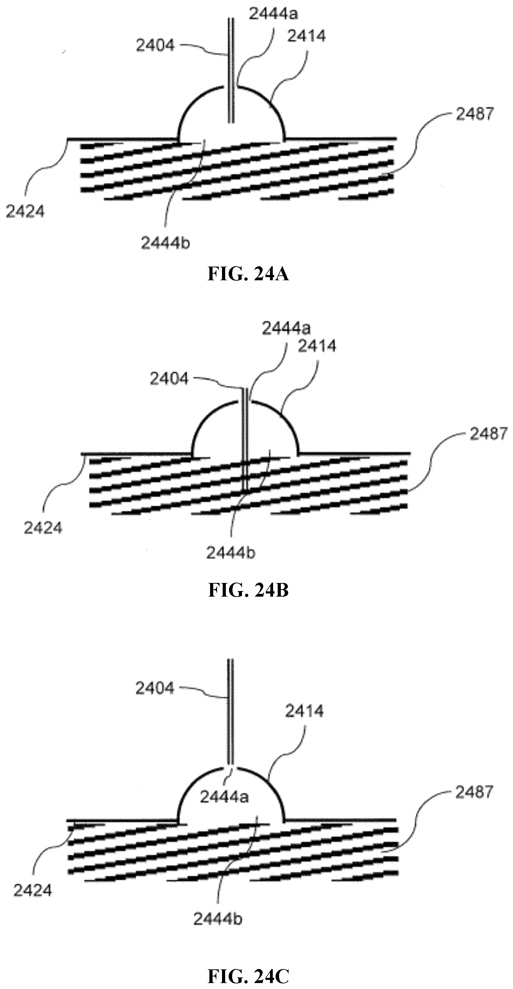

*FIGs. 24A-24C* are schematic illustrations of a needle safeguarding mechanism in accordance with an embodiment of the current invention. In some embodiment, a base includes a skin contact surface **2424.** Optionally the base may also include an indentation **2414.** Optionally, in some states a needle **2404** may be completely retracted into a housing of the device. For example, as illustrated in *FIG. 24C**,* needle **2404** may be retracted behind base **2424** including indentation **2414.** Optionally in an intermediate state, needle **2404** may project out from the inside of the housing to outside the housing through an opening **2444a** of the base, but remain protected inside indentation **2414** on the outside of the housing, for example as illustrated in *FIG. 24A**.* Optionally, in a fully extended state (for example as illustrated in *FIG. 24B**,* the needle may extend out beyond skin contact surface **2424** and into a skin **2487** of a subject.

In some embodiments, an injector may have a preactivated and/or a primed state with needle **2404** in an intermediate position (for example as illustrated in *FIG. 24A*)*.* For example, in a preactivated state, a tip of needle **2404** may be protected inside indentation **2414.** Optionally in the preactivated state the needle tip may be further protected by an adhesive cover that covers the gap in skin contact surface **2424** over indentation **2414.** Alternately or additionally, a needle may initially be covered by another needle cap. For example, a conventional tubular needle cover may be positioned over the needle. Optionally, the needle cap may be pulled off before use. Optionally the device is primed by removing the adhesive cover and/or needle cap and placing contact surface **2424** against skin **2487.**

In some embodiments, the needle may be extended out of indentation **2414** into a subject in an activated state (for example as illustrated in *FIG. 24B*)*.* For example, while the needle **2404** remains inside skin **2487,** needle **2404** may be used to inject a drug into the subject.

In some embodiments, at the end of the activated state, needle **2404** may be retracted. For example, when drug discharge ends and/or when the device is removed from skin **2487,** needle **2404** is retracted to a fully retracted position, for example as illustrated in *FIG. 24C**.*

In some embodiments, needle **2404** may be in a fully retracted position before injection. In some embodiments, needle **2404** may be retracted to an intermediate position after injection.

In various embodiments, an indentation may have various geometries. For example an indentation may be semi-spherical and/or conical and/or semi ovoid and/or irregular in shape. For example an indentation may have one or more axis of symmetry. In some embodiments, an indentation may intersect an edge of the device and/or be open one or more sides. Optionally the depth of an indentation (for example the height of indentation **2414** in the direction of the axis of needle **2404)** may range between 0.1 mm to 1 mm and/or 1 mm to 3 mm and/or 3mm to 8 mm and/or more than 8mm. The a width of an opening **2444b** of indentation **2414** at a skin contact surface may range for example between 0.1 to 1 mm and/or between 1 to 5mm and/or between 5 to 10 mm and/or from 10 to 30 mm and/or more. The a width of a needle hole (for example opening **2444a** and/or **944)** may range for example between .1 to 1 mm and/or between 1 to 5mm and/or between 5 to 10 mm and/or from 10 to 30 mm and/or more.

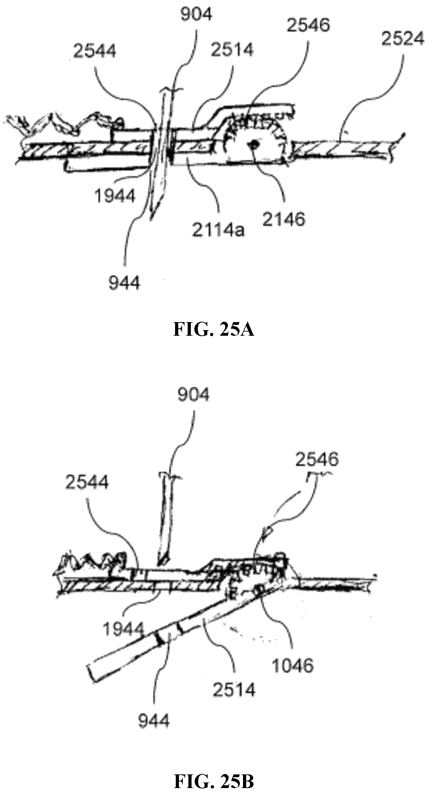

*FIGs. 25A-25B* are schematic illustrations of a rack and pinion needle safeguarding mechanism in accordance with an embodiment of the present invention. Optionally, during injection an opening **2544** in a sliding shield **2514** is aligned with an opening **1944** in a base **2524** of a device. For example, a rack and pinion system **2546,** optionally including a one way ratchet, slides shield **2514** when a pivoting shield **2514** opens (for example when the device is removed from the skin of a user) shield **2514** slides dis-aligning openings **1944** and **2544** and/or locking needle **904** into a housing. In some embodiments a rack and pinion system may unlock a needle before deployment and/or close onto (to bend and/or twist and/or break a needle after drug discharge).

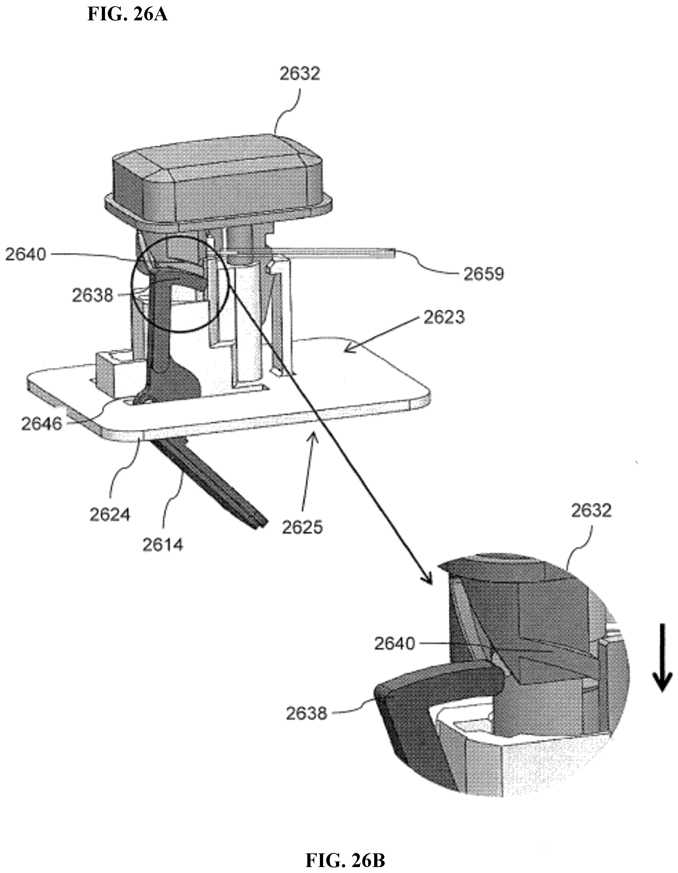

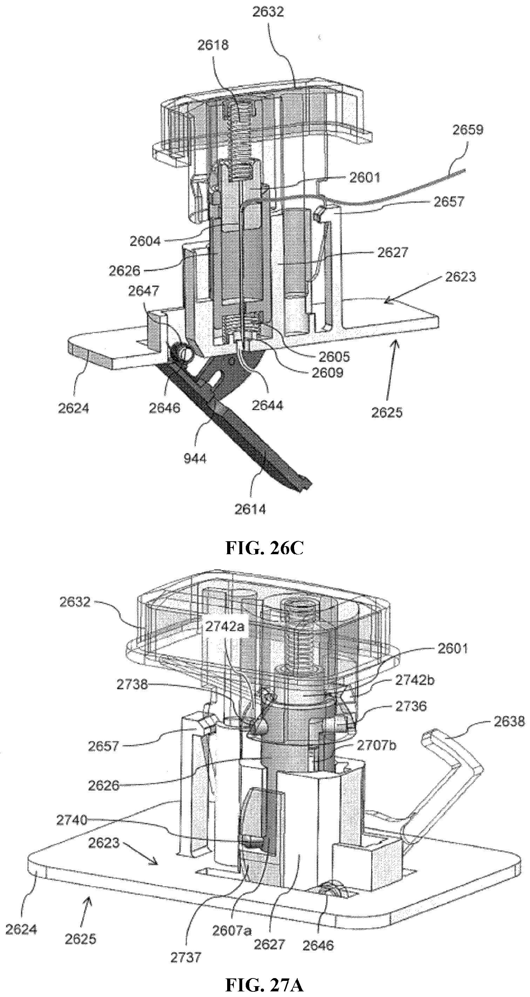

*FIGs. 26A, 26B* *and* *26C* illustrate an exemplary embodiment of a needle mechanism in accordance with an embodiment of the present invention. Optionally a needle mechanism has redundant protection. For example, a needle shield may protect a needle tip from becoming a stick hazard. Alternatively or additionally, the needle tip may be retracted to a protected location. Alternatively or additionally, a sensor may prevent extension of needle until the device has been properly primed and/or positioned on an injection zone. Alternatively or additionally, a needle shield may protect (for example by shielding) the needle tip when the needle is extended. Alternatively or additionally, the needle tip may be retracted to a protected location when the needle shield is compromised. In some embodiments, a single part may serve multiple functions. For example, a needle shield may also serve as a sensor for facilitating extension and/or retraction of the needle.

*FIG. 26A* is a perspective view of a needle mechanism in a preactivated locked state in accordance with an embodiment of the present invention (optionally as described for example in *FIG. 6* state **622).** Optionally a hollow needle **2604** (for example as illustrated in *FIG. 26C*) is connected on one end to a drug reservoir (for example by a flexible tube **2659** alternatively or additionally a needle may be directly attached to a reservoir for example to a syringe). The needle **2604** optionally includes a sharp tip **2609** at an end opposite from the reservoir.

In some embodiments, depressing an activation button **2632** causes needle tip **2609** to extend out of the device. For example, when the device is in the preactivated state and/or when the device is attached to the skin of a subject the activation. For example, activation may include the needle tip **2609** piercing the skin of a subject and/or the hollow of needle **2604** may form a fluid pathway between the drug reservoir and the subject.

In some embodiments, in the preactivated, locked state, tip **2609** is shielded by a housing of the device. For example, tip **2609** is supported on an inner side **2623** of a wall (for example base **2624)** of the housing. Optionally a safety latch **2638** of a skin sensor **2614** locks button **2632** in an un-depressed position inhibiting premature extension of tip **2609** and/or preventing a stick hazard. For example, when skin sensor **2614** is extended out from an outer surface **2625** of base **2624,** latch **2638** may block movement of an interference element **2640** and/or latch **2638** may block depressing of button **2632.**

*FIG. 26B* is a perspective view of a needle mechanism in a primed unlocked state in accordance with an embodiment of the present invention (optionally as described for example in *FIG. 6* state **614).** In some embodiments, when the outer surface **2625** of base **2624** is pushed against the skin of a subject (for example at an injection site), sensor **2614** is pushed upwards towards base **2624.** Optionally, pushing sensor **2614** towards base **2624** causes sensor **2614** and/or latch **2638** to pivot around a hinge **2646.** Pivoting optionally moves latch **2638** out of the way of interference element **2640** and/or facilitates depressing button **2632.**

In some embodiments, depressing button **2632** triggers an automatic needle insertions mechanism for example as illustrated in *FIG. 26C* to *FIG. 28C**.* Alternatively or additionally, force on button **2632** directly pushes needle **2626.** For example, needle **2604** may be rigidly attached to button **2632** and/or move therewith.

*FIG. 26C* is a cutaway view of a needle mechanism in accordance with an embodiment of the current invention. In some embodiments, a needle mechanism may include one or more stored energy sources. Stored energy may be used for needle extension, protection and/or for sensing. Optionally, one or more components of a needle retraction system will serve different functions depending on the state of the system. Optionally, the needle retraction system movable mounts needle **2604** a housing (for example a base **2624).** For example, the needle mechanism may movably connect needle **2604** to base**2624**. For example, a tip **2609** of a needle **2604** may move longitudinally with respect to the housing. For example, a sharp tip **2609** may move through a needle hole **2644** to be exposed and/or protected, for example as described herein below.

In some embodiments, needle extension, retraction and/or shielding may be driven by a stored energy source. For example, a compression spring **2618** may drive the needle extension. For example, a second counter compression spring **2605** may drive needle retraction. For example, a torsion spring **2647** may drive deployment of a skin sensor **2614.** Optionally sensor **2614** may serve multiple functions at different states of the system. For example, sensor **2614** may act as a skin sensor to unlock needle extension when the device is placed on an injection site. For example, sensor **2614** may act as a needle shield protecting a sharp needle point after the end of drug delivery and/or while the needle is extended. For example, sensor **2614** may act as a trigger needle for retraction. Optionally, a single stored energy device may drive multiple movements. For example, a single torsion spring may drive a wheel wherein a first half turn causes needle extension and a second half turn causes needle retraction and/or a compression spring may pull a pin along a track which moves the needle first to the extended position and subsequently back to the retracted position.

In some embodiments, sensor **2614** may be mechanically interlocked with a needle extension mechanism (for example button **2632** as illustrated for example in *FIGs. 26A and 26B*). For example, the interlock may unlock a needle extension mechanism when the injector in a locked preprimed state is placed on an injection site, for example as explained in reference to *FIGs. 26A and 26B**.*

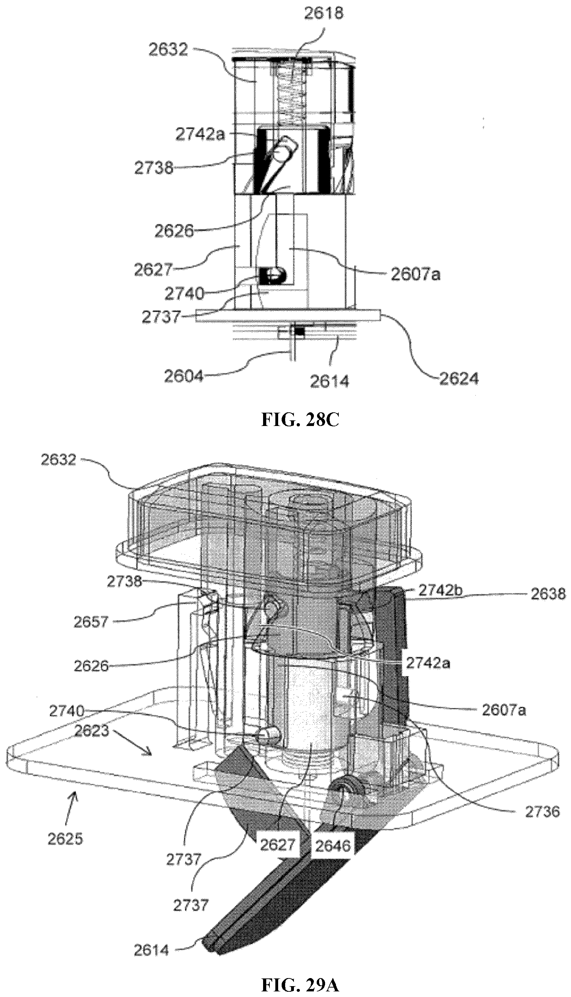

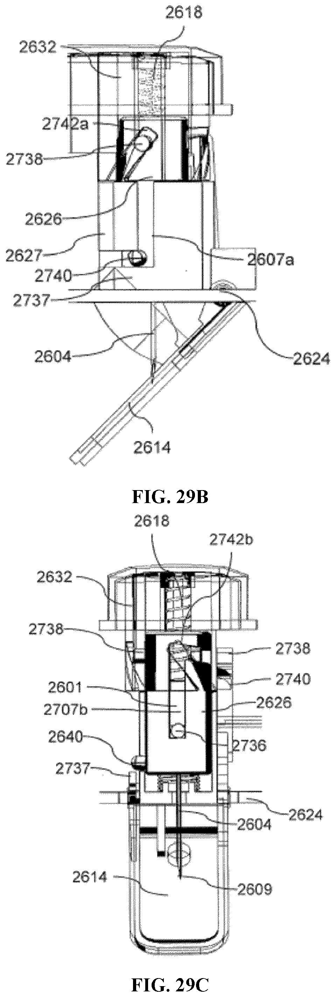

In some embodiments, sensor **2614** may shield a needle. For example, after drug delivery and/or when the delivery device is removed from the injection site, sensor **2614** may be deployed to shield a needle (as illustrated for example in *FIGs 29A-29C* and/or in connection to a shielded state in *FIG. 5* and/or in connection to a partially protected state and/or in connection to *FIGs. 13A-13C*).

*FIGs. 27A to 27C* illustrate a needle assembly in a primed state in accordance with an embodiment of the current invention. In some embodiments, a skin contact surface on an outer surface **2625** of the injector is adhered to the skin of a subject. Optionally, placing the skin contact surface onto the skin forces pushes sensor **2614** and/or pivots it upward around pivot **2646.** Optionally, sensor against moves upward until is rests against and/or is flush to outer surface **2624.** Optionally, as sensor **2614** pivots upward, safety latch **2638** pivots away from activation button **2632,** freeing button **2632** to move downward. Optionally, freeing button **2632** to move downward, primes the device.

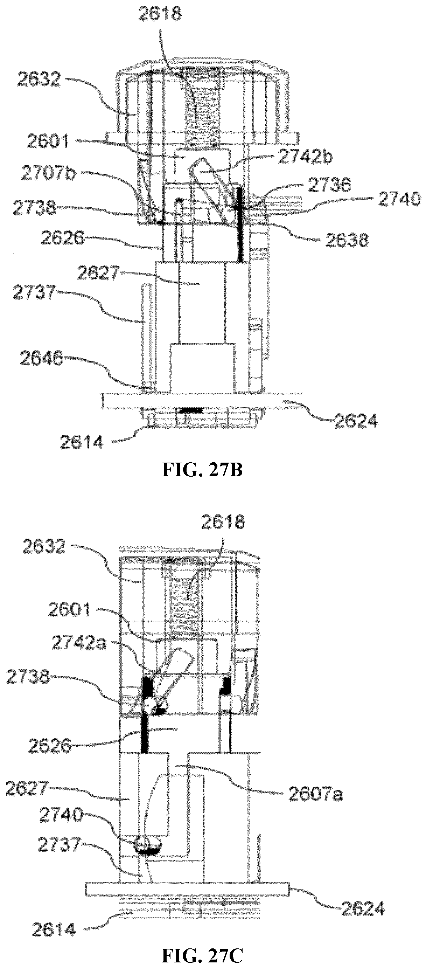

*FIG. 27B* illustrates a needle insertion mechanism in a primed state in accordance with an embodiment of the present invention. In some embodiments, needle insertion may be powered by stored energy in the device. For example, spring **2618** pushes downward on a needle holder **2601** rigidly connected to the needle **2604.** In the primed state, the needle **2604 is** optionally locked in a retracted position by a pin **2736** in a track **2707b.** A slanted track **2742b** in button **2632** optionally controls the horizontal position of pin **2736.** As button **2632** is depressed (vertically downward) pin **2736** is optionally forced by slanted track **2742b** leftward (e.g. by rotating needle holder **2601).**

*FIG. 27C* illustrates a needle retraction mechanism in a primed state in accordance with an embodiment of the present invention. In some embodiments, needle retraction may be powered by stored energy in the device. For example, spring **2605** (for example as illustrated in *FIG. 26C*) pushes upward on a retraction slider **2626.** Slider **2626** optionally includes two pins **2738** and **2740.** In the primed state, slider **2626** is optionally locked in a pre-retracted position by a pin **2740** in a track **2707a.** A slanted track **2742b** in button **2632** optionally controls the horizontal position of pin **2736.** As button **2632** is depressed (vertically downward) a second pin **2738** is optionally forced by slanted track **2742a** rightward (rotating slider **2626** and/or pin **2740** rightward).

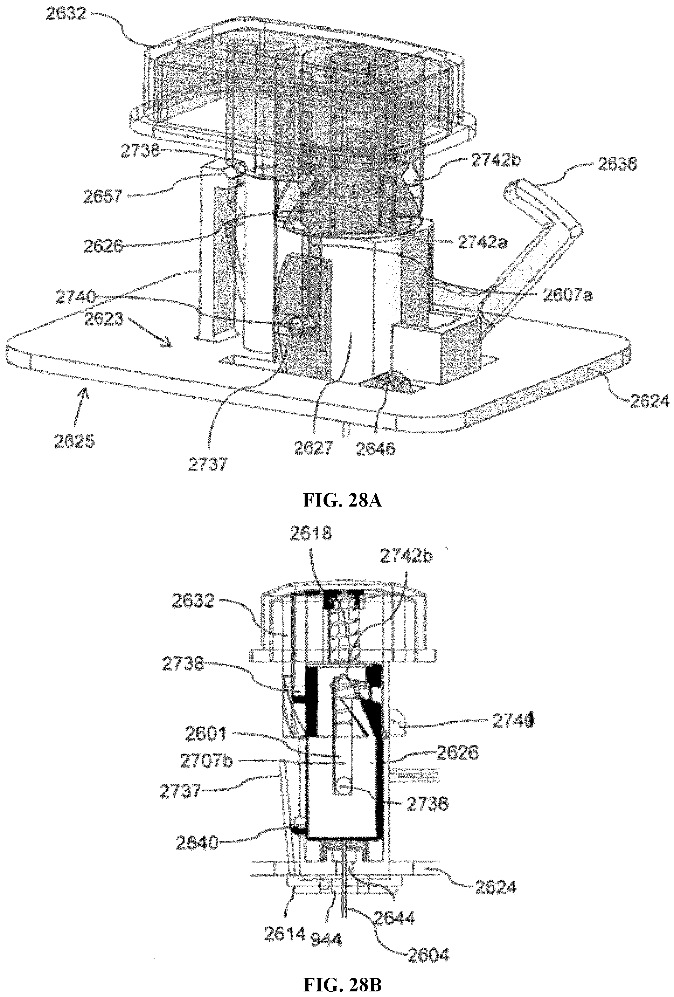

*FIGs. 28A to 28C* illustrate a needle assembly in an activated state in accordance with an embodiment of the current invention. In some embodiments, a user depresses an activation button **2632** to activate the device. For example, as illustrated in *FIG. 28B**,* when button **2632** is fully depressed, track **2742b** has forced pin **2736** leftward until it has reached a vertical portion of track **2707b.** In some embodiments, once pin reaches the vertical portion of track **2707b,** it is free to move downward, unlocking downward movement of needle holder **2601.** Optionally, once unlocked needle holder **2601** and needle **2604** are pushed downward by expanding spring **2618.** Downward movement optionally extends sharp tip **2609** (illustrated for example in *FIG. 26C*) of needle **2604** out openings **944** and **2644** in base **2624** and/or shield **2614** respectively.

In some embodiments, needle tip **2609** is extended out of opening **2644** of base **2624** and/or opening **944** of sensor **2614** while outer surface **2625** of base **2624** is adhered to a skin of a subject. Optionally, an opposite end of needle **2604** remains in fluid connection with a medicine reservoir inside the housing of the delivery device. For example the hollow of needle **2604** becomes a fluid path for injecting a drug from the reservoir into the subject.

*FIG. 28C* illustrates a needle retraction mechanism in an active state in accordance with an embodiment of the current invention. Optionally, when the device is activated (for example by depressing activation button **2632)** the retraction mechanism is moved to an armed mode, ready to retract. For example, pin **2740** is moved close to its unlocked position, the vertical portion of track **2707a.** Alternatively or additionally, a retraction mechanism may be armed and/or further armed to a ready to retract configuration when the device is removed from the skin for example when skin sensor **2614** is deployed (for example as illustrated in *FIGs. 29A-C*).

In some embodiments, a lock of the retraction mechanism is a mechanically linked to skin sensor **2614.** For example, pin **2740** interferes with an arm **2737** of skin sensor **2614.** For example, in *FIG. 28B* it is seen that in the active state, pin **2640** contacts a arm **2737** of sensor **2614** and/or elastically deforms arm **2737** outward.

*FIGs. 29A-29C* illustrate a needle retraction mechanism in a partially protected state in accordance with an embodiment of the current invention (for example as described in state **605** of *FIG. 6* and/or step **308** of *FIG. 3*). Optionally, in the partially protected state, needle **2604** remains in an extended state and/or sensor **2614** is deployed to shield sharp tip **2609.** Optionally, a skin sensor is deployed when a delivery device is removed from delivery site. For example, sensor **2614** pivots around pivot **2646** such that sensor **2614** covers the sharp tip **2609** that passes through aperture **944** which is moved out of alignment with sharp tip **2609.**

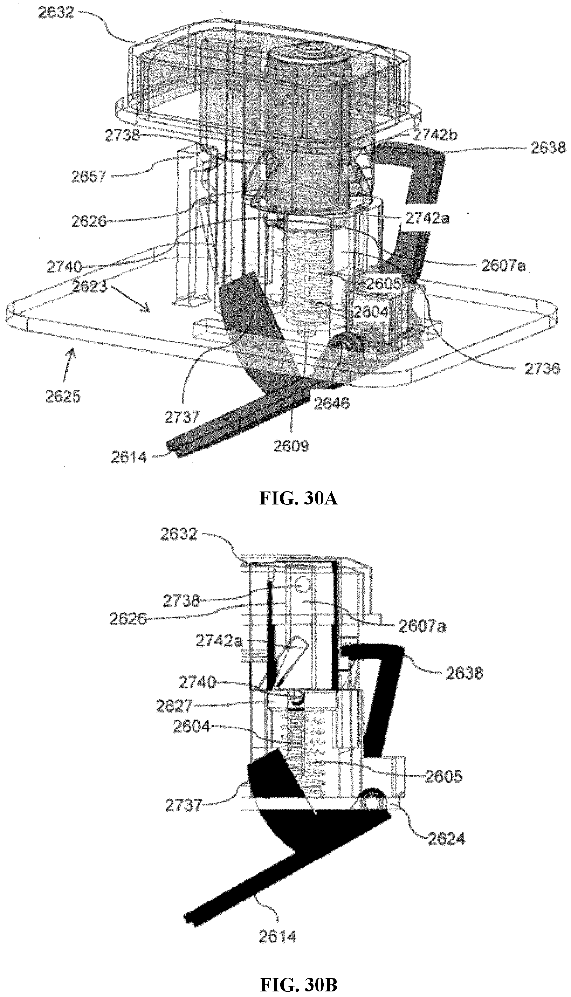

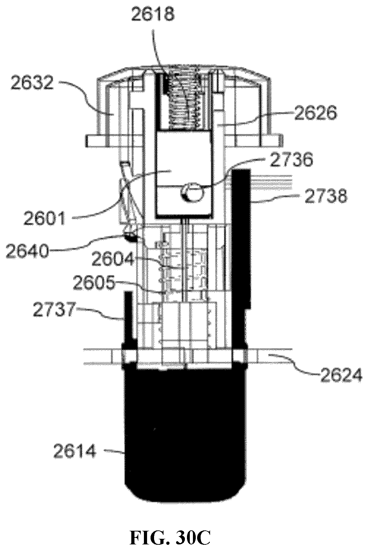

*FIGs. 30A-30C* illustrate a needle retraction mechanism in a fully protected state in accordance with an embodiment of the current invention (for example as described in state **604** of *FIG. 6* and/or step **305** of *FIG. 3*). Optionally, in the fully protected state sharp tip **2609** has retraced behind outer surface **2625** (for example into opening **2644).** Alternatively or additionally, in the fully protected state a sharp tip retracts behind the surface **2625.**

In some embodiments, when a needle shield is compromised, a needle is retracted. For example, when skin sensor **2614** is moved from the partially protected state shielding sharp tip **2609** towards housing **2624** (for example, pivoting upward around pivot **2646)** arm **2737** pushes pin **2740** into the vertical portion of track **2707a** unlocking needle retraction slider **2626.** When slider **2626** is unlocked, it is optionally moved automatically away from opening **2644.** For example, spring **2605** expands pushing up slider **2626,** needle holder **2601** and/or needle **2604.** Optionally, the upward movement also moves sharp tip **2609** into the retracted position and/or a fully protected state. Alternatively or additionally, retraction may not be automatic. For example retraction may be driven by a force, for example an upward force of sensor **2614** on needle tip **2609** and/or holder **2601** and/or slider **2626.**

It is expected that during the life of a patent maturing from this application many relevant technologies will be developed and the scope of the terms are intended to include all such new technologies *a priori.*

As used herein the term "about" refers to ±5%.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## Claims

1. A needle insertion and retraction mechanism for an injector having a housing (724) with a base defining an opening, the needle insertion and retraction mechanism comprising:
an injection needle (904) mounted to a needle retainer (901), the needle retainer (901) being linearly slidable along a first track (907) between a retracted position, in which a tip of the injection needle (904) is within the housing (724), and an extended position, in which the tip of the injection needle (904) projects outward from the housing (724) through the opening;
a shield (914) defining a needle aperture (944) and having an interference element (1038) extending therefrom, the shield (914) being movable relative to the housing (724) between a deployed position, in which the needle aperture (944) is misaligned with the injection needle (904) and a withdrawn position, in which the needle aperture (944) is aligned with the injection needle (904);
a retraction spring (905) engaged with the needle retainer (901) at one end and engaged with the shield (914) at an opposing end;
a driver (926) engageable with the needle retainer (901), the driver (926) being linearly slidable along, and rotatable about, a second track (2742b), the driver (926) being slidable by a main spring (918), the retraction spring (905) and the main spring (918) providing biasing forces in opposing directions; and
an activation button (1032) depressible to rotate the driver (926) about the second track (2742b, 927) into engagement with needle mount (1130);
the needle insertion and retraction mechanism being **characterized in that**:
in a pre-activation state of the insertion and retraction mechanism before the injector is placed on a skin surface of a patient, the biasing force of the retraction spring (905) orients the shield (914) in the deployed position, in which the interference element (1038) blocks downward movement of the activation button (1032), locking the needle retainer (901) and the injection needle (904) in the retracted state, and the retraction spring (905) is in an expanded configuration and the main spring (918) is in a compressed configuration;
in a primed state of the insertion and retraction mechanism, the injector is placed on the skin surface of the patient and the shield (914) is moved to the withdrawn position thereof, in which the retraction spring (905) is partially compressed and the interference element (1038) is moved out of the way of the activation button (1032);
in an activation state of the insertion and retraction mechanism, the activation button (1032) is depressed, engaging the driver (926) with the needle mount (1130) and freeing the main spring (918) to push the driver (926) and the engaged needle retainer (901) and the injection needle (904) into the extended position, thereby further compressing the retraction spring (905); and
in an intermediate state of the insertion and retraction mechanism after the injector is removed from the skin surface of the patient, the driver (926) is disengaged from the needle mount (1130), and the retraction spring (905) partially expands and returns the shield (914) to the deployed state, in which the shield (914) restricts the needle mount (1130) from returning to the retracted position and covers the tip of the injection needle (904), and wherein subsequent contact with the shield (914) toward the withdrawn position releases the needle mount (1130), whereby the retraction spring (905) further expands and retracts the needle mount (1130) and injection needle (904).

2. The mechanism of claim 1, wherein the tip of the injection needle (904) is positioned between said shield (914) and said housing (724) in the intermediate state.

3. The mechanism of claim 1, wherein said contact with the shield (914) toward the withdrawn position in the intermediate state comprises exerting a force of at least 1 N on said shield (914) toward said housing (724).

4. The mechanism of any of claims 1-3, wherein said shield (914) is pivotally mounted on said housing (724), in the deployed position, said shield (914) is pivoted away from said housing (724), and in the withdrawn position, said shield (914) is flush with said housing (724).

5. The mechanism of any of claims 1-4, wherein the retraction spring (905) maintains the needle retainer (901) in the retracted position at one end and the shield (914) in the deployed position at the other end, in a fully expanded configuration of the retraction spring (905).

6. The mechanism of any of claims 1-5, further comprising a blocking element (942) and wherein the driver (926) includes a protrusion (1036), and wherein, in the compressed configuration of the main spring (918), the protrusion (1036) of the driver (926) is abutted by the blocking element (942), maintaining the main spring in the compressed configuration and inhibiting downward movement of driver (926).

7. The mechanism of claim 6, wherein the activation button (1032) includes a wedge element (1034) configured to transform a force in a downward direction on the button to a force in a sideways direction on the driver (926).

8. The mechanism of claim 7, wherein depressing the activation button (1032) in the activation state pushes the wedge element (1034) against the protrusion (1036) of the driver (926) and transmits a lateral force onto the protrusion (1036), causing a torque on the driver which rotates the driver (926) around the second track, disengaging the protrusion (1036) from the blocking element (942) and freeing the main spring (918).

9. The mechanism of claim 8, wherein the driver (926) comprises an element and the needle mount comprises a corresponding interference element (1130), and wherein rotating the driver (926) linearly aligns the element of the driver (926) with the corresponding interference element (1130) of needle retainer (901), thereby linking linear downward movement of the driver (926) and the needle retainer (901).

## Patentansprüche

1. Nadeleinführungs- und -rückzugmechanismus für einen Injektor, der ein Gehäuse (724) mit einer Basis hat, die eine Öffnung definiert, wobei der Nadeleinführungs- und - rückzugmechanismus umfasst:
eine Injektionsnadel (904), die an einem Nadelhalter montiert ist (901), wobei der Nadelhalter (901) entlang einer ersten Bahn (907) zwischen einer eingezogenen Stellung, in der eine Spitze der Injektionsnadel (904) innerhalb des Gehäuses (724) ist, und einer ausgezogenen Stellung, in der die Spitze der Injektionsnadel (904) aus dem Gehäuse (724) durch die Öffnung herausragt, linear verschiebbar ist;
einen Schutz (914), der eine Nadelöffnung (944) definiert und ein Interferenzelement (1038) hat, das sich daraus erstreckt, wobei der Schutz (914) relativ zum Gehäuse (724) zwischen einer Einsatzstellung, in der die Nadelöffnung (944) zur Injektionsnadel (904) fehlausgerichtet ist, und einer zurückgezogenen Stellung, in der die Nadelöffnung (944) zur Injektionsnadel (904) ausgerichtet ist, beweglich ist;
eine Rückziehfeder (905), die in den Nadelhalter (901) an einem Ende eingreift und in den Schutz (914) an einem entgegengesetzten Ende eingreift;
einen Treiber (926), der in den Nadelhalter (901) in Eingriff bringbar ist, wobei der Treiber (926) entlang einer zweiten Bahn (2742b) linear verschiebbar ist und um dieselbe drehbar ist, der Treiber (926) mittels einer Hauptfeder (918) verschiebbar ist, die Rückziehfeder (905) und die Hauptfeder (918) Vorspannkräfte in entgegengesetzte Richtungen aufbringen; und
einen Aktivierungsknopf (1032), der niederdrückbar ist, um den Treiber (926) um die zweite Bahn (2742b, 927) in einen Eingriff mit der Nadelhalterung (1130) zu drehen;
wobei der Nadeleinführungs- und -rückzugmechanismus **dadurch gekennzeichnet ist, dass**:
in einem Voraktivierungszustand des Einführungs- und Rückzugmechanismus, bevor der Injektor auf eine Hautoberfläche eines Patienten gestellt wird, die Vorspannkraft der Rückziehfeder (905) den Schutz (914) in die Einsatzstellung richtet, in der das Interferenzelement (1038) die Bewegung nach unten des Aktivierungsknopfes (1032) sperrt, wodurch der Nadelhalter (901) und die Injektionsnadel (904) im eingezogenen Zustand verriegelt werden, und die Rückziehfeder (905) in einer ausgeweiteten Konfiguration ist und die Hauptfeder (918) in einem zusammengedrückten Konfiguration ist;
in einem vorbereiteten Zustand des Einführungs- und Rückzugmechanismus, der Injektor auf die Hautoberfläche des Patienten gestellt wird und der Schutz (914) zur zurückgezogenen Stellung davon bewegt wird, in der die Rückziehfeder (905) teilweise zusammengedrückt wird und das Interferenzelement (1038) weg vom Pfad des Aktivierungsknopfes (1032) bewegt wird;
in einem Aktivierungszustand des Einführungs- und Rückzugmechanismus, der Aktivierungsknopf (1032) niedergedrückt ist, wodurch der Treiber (926) mit der Nadelhalterung (1130) in Eingriff gebracht wird und die Hauptfeder (918) freigegeben wird, um den Treiber (926) und den eingegriffenen Nadelhalter (901) und die Injektionsnadel (904) in die ausgezogene Stellung zu drücken, wodurch die Rückziehfeder (905) ferner zusammengedrückt wird; und
in einem Zwischenzustand des Einführungs- und Rückzugmechanismus, nachdem der Injektor von der Hautoberfläche des Patienten entfernt wird, der Treiber (926) von der Nadelhalterung (1130) gelöst wird, und die Rückziehfeder (905) sich teilweise ausdehnt und den Schutz (914) zum Einsatzzustand zurückbringt, in dem der Schutz (914) die Nadelhalterung (1130) daran hindert, zur eingezogenen Stellung zurückzukehren, und die Spitze der Injektionsnadel (904) deckt, und worin der anschließende Kontakt mit dem Schutz (914) zur zurückgezogenen Stellung die Nadelhalterung (1130) freilässt, wobei die Rückziehfeder (905) sich weiter ausdehnt und die Nadelhalterung (1130) und die Injektionsnadel (904) zurückzieht.

2. Mechanismus nach Anspruch 1, worin die Spitze der Injektionsnadel (904) zwischen dem Schutz (914) und dem Gehäuse (724) im Zwischenzustand positioniert ist.

3. Mechanismus nach Anspruch 1, worin der Kontakt mit dem Schutz (914) zur zurückgezogenen Stellung im Zwischenzustand das Ausüben einer Kraft von zumindest 1 N auf den Schutz (914) zum Gehäuse (724) hin umfasst.

4. Mechanismus nach einem der Ansprüche 1-3, worin der Schutz (914) am Gehäuse (724), in der Einsatzstellung, schwenkbar montiert ist, der Schutz (914) vom Gehäuse (724) weggeschwenkt ist, und in der zurückgezogenen Stellung der Schutz (914) mit dem Gehäuse (724) bündig ist.

5. Mechanismus nach einem der Ansprüche 1-4, worin die Rückziehfeder (905) den Nadelhalter (901) in der eingezogenen Stellung an einem Ende und den Schutz (914) in der Einsatzstellung am anderen Ende, in einer völlig ausgedehnten Konfiguration der Rückziehfeder (905), hält.

6. Mechanismus nach einem der Ansprüche 1-5, ferner umfassend ein Sperrelement (942) und worin der Treiber (926) einen Vorsprung (1036) umfasst, und worin, in der zusammengedrückten Konfiguration der Hauptfeder (918), der Vorsprung (1036) des Treibers (926) vom Sperrelement (942) angestoßen wird, wodurch die Hauptfeder in der zusammengedrückten Konfiguration gehalten wird und die Bewegung nach unten des Treibers (926) verhindert wird.

7. Mechanismus nach Anspruch 6, worin the Aktivierungsknopf (1032) ein Keilelement (1034) umfasst, das dafür ausgelegt ist, eine Kraft in einer Abwärtsrichtung auf den Knopf in eine Kraft in einer seitlichen Richtung auf den Treiber (926) umzuwandeln.

8. Mechanismus nach Anspruch 7, worin das Niederdrücken des Aktivierungsknopfes (1032) im Aktivierungszustand das Keilelement (1034) gegen den Vorsprung (1036) des Treibers (926) drückt und eine seitliche Kraft auf den Vorsprung (1036) überträgt, was ein Drehmoment am Treiber bewirkt, das den Treiber (926) um die zweite Bahn dreht, wodurch der Vorsprung (1036) vom Sperrelement (942) löst und die Hauptfeder (918) freilässt.

9. Mechanismus nach Anspruch 8, worin der Treiber (926) ein Element umfasst und die Nadelhalterung ein entsprechendes Interferenzelement (1130) umfasst, und worin das Drehen des Treibers (926) das Element des Treibers (926) zu dem entsprechenden Interferenzelement (1130) des Nadelhalters (901) linear ausrichtet, wodurch die lineare Bewegung nach unten des Treibers (926) und der Nadelhalter (901) verknüpft werden.

## Revendications

1. Mécanisme d'insertion et de rétraction d'aiguille pour un injecteur présentant un boîtier (724) doté d'une base définissant une ouverture, le mécanisme d'insertion et de rétraction d'aiguille comprenant :
une aiguille d'injection (904) montée sur un organe de retenue d'aiguille (901), l'organe de retenue d'aiguille (901) étant apte à coulisser linéairement le long d'une première glissière (907) entre une position rétractée, dans laquelle une pointe de l'aiguille d'injection (904) se trouve à l'intérieur du boîtier (724), et une position étendue, dans laquelle la pointe de l'aiguille d'injection (904) fait saillie vers l'extérieur du boîtier (724) par l'ouverture ;
un écran (914) définissant un orifice d'aiguille (944) et présentant un élément d'interférence (1038) s'étendant à partir de cet écran, l'écran (914) étant mobile par rapport au boîtier (724) entre une position déployée, dans laquelle l'orifice d'aiguille (944) est désaligné par rapport à l'aiguille d'injection (904) et une position retirée, dans laquelle l'orifice d'aiguille (944) est aligné avec l'aiguille d'injection (904) ;
un ressort de rétraction (905) en prise avec l'organe de retenue d'aiguille (901) à une extrémité et en prise avec l'écran (914) à une extrémité opposée ;
un organe d'entraînement (926) apte à venir en prise avec l'organe de retenue d'aiguille (901), l'organe d'entraînement (926) étant apte à coulisser linéairement le long d'une deuxième glissière (2742b) et à tourner autour de celle-ci, l'organe d'entraînement (926) étant apte à coulisser sous l'action d'un ressort principal (918), le ressort de rétraction (905) et le ressort principal (918) fournissant des forces de sollicitation dans des directions opposées ; et
un bouton d'activation (1032) enfonçable pour faire tourner l'organe d'entraînement (926) autour de la deuxième glissière (2742b, 927) jusqu'à mettre l'organe d'entraînement (926) en prise avec un support d'aiguille (1130) ;
le mécanisme d'insertion et de rétraction d'aiguille étant **caractérisé en ce que** :
dans un état de pré-activation du mécanisme d'insertion et de rétraction avant que l'injecteur ne soit placé sur une surface cutanée d'un patient, la force de sollicitation du ressort de rétraction (905) oriente l'écran (914) dans la position déployée, dans laquelle l'élément d'interférence (1038) bloque le déplacement descendant du bouton d'activation (1032), verrouillant l'organe de retenue d'aiguille (901) et l'aiguille d'injection (904) dans l'état rétracté, et le ressort de rétraction (905) est dans une configuration allongée et le ressort principal (918) est dans une configuration comprimée ;
dans un état amorcé du mécanisme d'insertion et de rétraction, l'injecteur est placé sur la surface cutanée du patient et l'écran (914) est déplacé dans sa position retirée, dans laquelle le ressort de rétraction (905) est partiellement comprimé et l'élément d'interférence (1038) est déplacé hors du passage du bouton d'activation (1032) ;
dans un état d'activation du mécanisme d'insertion et de rétraction, le bouton d'activation (1032) est enfoncé, mettant en prise l'organe d'entraînement (926) avec le support d'aiguille (1130) et libérant le ressort principal (918) pour qu'il pousse l'organe d'entraînement (926) et l'organe de retenue d'aiguille (901) mis en prise et l'aiguille d'injection (904) dans la position étendue, comprimant ainsi davantage le ressort de rétraction (905) ; et
dans un état intermédiaire du mécanisme d'insertion et de rétraction après que l'injecteur a été enlevé de la surface cutanée du patient, l'organe d'entraînement (926) est désengagé du support d'aiguille (1130), et le ressort de rétraction (905) s'allonge partiellement et ramène l'écran (914) dans l'état déployé, dans lequel l'écran (914) restreint le retour du support d'aiguille (1130) dans la position rétractée et couvre la pointe de l'aiguille d'injection (904), et dans lequel un contact ultérieur avec l'écran (914) vers la position retirée libère le support d'aiguille (1130), de sorte que le ressort de rétraction (905) s'allonge davantage et rétracte le support d'aiguille (1130) et l'aiguille d'injection (904).

2. Mécanisme selon la revendication 1, dans lequel la pointe de l'aiguille d'injection (904) est positionnée entre ledit écran (914) et ledit boîtier (724) dans l'état intermédiaire.

3. Mécanisme selon la revendication 1, dans lequel ledit contact avec l'écran (914) vers la position retirée dans l'état intermédiaire comprend le fait d'exercer une force d'au moins 1 N sur ledit écran (914) vers ledit boîtier (724).

4. Mécanisme selon l'une quelconque des revendications 1 à 3, dans lequel ledit écran (914) est monté de manière pivotante sur ledit boîtier (724), dans la position déployée, ledit écran (914) est écarté, par pivotement, dudit boîtier (724), et, dans la position retirée, ledit écran (914) affleure ledit boîtier (724).

5. Mécanisme selon l'une quelconque des revendications 1 à 4, dans lequel le ressort de rétraction (905) maintient l'organe de retenue d'aiguille (901) dans la position rétractée à une extrémité et l'écran (914) dans la position déployée à l'autre extrémité, dans une configuration complètement allongée du ressort de rétraction (905).

6. Mécanisme selon l'une quelconque des revendications 1 à 5, comprenant en outre un élément de blocage (942) et dans lequel l'organe d'entraînement (926) comporte une saillie (1036), et dans lequel, dans la configuration comprimée du ressort principal (918), la saillie (1036) de l'organe d'entraînement (926) est en butée contre l'élément de blocage (942), maintenant le ressort principal dans la configuration comprimée et inhibant le déplacement descendant de l'organe d'entraînement (926).

7. Mécanisme selon la revendication 6, dans lequel le bouton d'activation (1032) comporte un élément formant coin (1034) configuré pour transformer une force dans une direction descendante sur le bouton en une force dans une direction latérale sur l'organe d'entraînement (926).

8. Mécanisme selon la revendication 7, dans lequel le fait d'enfoncer le bouton d'activation (1032) dans l'état d'activation pousse l'élément formant coin (1034) contre la saillie (1036) de l'organe d'entraînement (926) et transmet une force latérale sur la saillie (1036), engendrant sur l'organe d'entraînement un couple qui fait tourner l'organe d'entraînement (926) autour de la deuxième glissière, désengageant la saillie (1036) de l'élément de blocage (942) et libérant le ressort principal (918).

9. Mécanisme selon la revendication 8, dans lequel l'organe d'entraînement (926) comprend un élément et le support d'aiguille comprend un élément d'interférence correspondant (1130), et dans lequel le fait de faire tourner l'organe d'entraînement (926) aligne linéairement l'élément de l'organe d'entraînement (926) avec l'élément d'interférence correspondant (1130) de l'organe de retenue d'aiguille (901), liant ainsi le déplacement linéaire descendant de l'organe d'entraînement (926) et de l'organe de retenue d'aiguille (901).
